# EUROPÄISCHE PATENTSCHRIFT

(11) **EP 1 073 426 B1**
(45) Veröffentlichungstag und Bekanntmachung des Hinweises auf die Patenterteilung: **03.03.2004**
(21) Anmeldenummer: 99923490.9
(22) Anmeldetag: 29.04.1999
(51) Int. Cl.: A61K 9/51, A61K 9/16, A61K 38/13

(54) **PHARMAZEUTISCHE CICLOSPORIN-FORMULIERUNG MIT VERBESSERTEN BIOPHARMAZEUTISCHEN EIGENSCHAFTEN, ERHÖHTER PHYSIKALISCHER QUALITÄT & STABILITÄT SOWIE VERFAHREN ZUR HERSTELLUNG**
PHARMACEUTICAL CYCLOSPORIN FORMULATION WITH IMPROVED BIOPHARMACEUTICAL PROPERTIES, IMPROVED PHYSICAL QUALITY AND GREATER STABILITY, AND METHOD FOR PRODUCING SAID FORMULATION
FORMULATION PHARMACEUTIQUE DE CICLOSPORINE PRESENTANT DE MEILLEURES PROPRIETES BIOPHARMACEUTIQUES, UNE MEILLEURE QUALITE ET STABILITE EN TERMES DE PHYSIQUE, ET PROCEDE PERMETTANT DE LA PREPARER

(30) Priorität: 30.04.1998 DE 19819273
(43) Veröffentlichungstag der Anmeldung: 07.02.2001
(73) Patentinhaber: PHARMATEC INTERNATIONAL S.r.l., 20098 San Giuliano Milanese (MI) (IT)
(72) Erfinder: PENKLER, Lawrence, John, Port Elizabeth 6070 (ZA); MÜLLER, Rainer, Helmut, D-12161 Berlin (DE); RUNGE, Stephan, Anton, D-13509 Berlin (DE); RAVELLI, Vittorino, I-20100 Mailand (IT)
(74) Vertreter: Weisgerber, Stefan
(86) Internationale Anmeldenummer: PCT/EP1999/002892
(87) Internationale Veröffentlichungsnummer: WO 1999/056733

(56) Entgegenhaltungen:
- WO-A-99/13864
- US-A- 5 430 021

## Beschreibung

Die Erfindung beschreibt mit Ciclosporin (gelegentlich auch Cyclosporin geschrieben) oder Ciclosporinderivaten natürlichen und/oder synthetischen Ursprungs beladene partikuläre Systeme mit verbesserten biopharmazeutischen Eigenschaften für Ciclosporine *in vivo,* verbesserter Qualität (Feinheit und Gleichförmigkeit. der Partikel, Arzneistoffeinschluß) und verbesserter physikalischer Stabilität der partikulären Formulierung (Ausbleiben von Aggregation und Gelbildung).

### Hintergrund der Erfindung

Ciclosporine sind cyclische Oligopeptide. Es handelt sich um eine Gruppe natürlicher Oligopeptide von Ciclosporin A bis Ciclosporin Z. Synthetische Derivate sind ebenfalls beschrieben worden (SDZ IMM 125, Hydroxyethyl Derivat des D-Serin-8-Ciclosporins).

Ciclosporin A ist ein lipophiles Molekül bestehend aus 11 Aminosäuren. Es wird durch Pilz-Fermentierung gewonnen. Das Molekulargewicht beträgt 1203.

Handelsprodukte: Sandimmun®, Sandimmun Optoral® (außerhalb Deutschlands = Sandimmun Neoral®) [A. Meinzer, E. Müller, J. Vonderscher, Perorale Mikroemulsionsformulierung - Sandimmun Optoral®/Neoral®, in: Pharmazeutische Technologie: Moderne Arzneiformen, R.H. Müller und G.E. Hildebrand (Hrsg.), Wissenschaftliche Verlagsgesellschaft Stuttgart, 169-177, 1998].

Ciclosporin A wird vorzugsweise als Immunsuppressivum nach Organtransplantationen eingesetzt. Andere Anwendungsgebiete sind Autoimmunerkrankungen, Psoriasis und Diabetes. Alle Ciclosporine - natürliche und synthetische - können in dieser Erfindung genutzt werden.

Die Ciclosporine sind sehr lipophile Stoffe und in Wasser nur sehr schwer löslich (z.B. Ciclosporin A: < 0,004% m/V in Wasser bei 25 °C). Die hohe Lipophilie und schlechte Wasserlöslichkeit sind die Hauptprobleme für die Herstellung einer geeigneten pharmazeutischen Zubereitung. Aufgrund der besseren Löslichkeit in fetten Ölen und Alkohol wurde Sandimmun' mit diesen Inhaltsstoffen als Lösungsvermittler für die orale Anwendung in Form eines Emulsionskonzentrates entwickelt. Das Emulsionskonzentrat besteht aus 100 mg Ciclosporin, gelöst in 1 ml einer Mischung aus Öl, Ethanol und einem Emulgator, Macrogolglycerol-trioleat-lino-lat. Das Konzentrat muß vor Gebrauch verdünnt werden, z.B. durch Einrühren mit einem Löffel in kalte Milch, Kakao oder in Fruchtsaft. Diese nicht-standardisierte, ineffiziente Mischungsprozedur führt zur Bildung einer groben, inhomogenen O/W Emulsion mit relativ großer Tröpfchengröße. In vivo variiert die Bioverfügbarkeit nach oraler Gabe im Extremfall zwischen 10% bis 60% [T. Beveridge, A., Gratwohl, F., Michot, et al., Curr. Ther. Res., 30 (5), 1981).

Neben der Formulierung als Trinklösung ist Sandimmun® auch als Kapsel erhältlich. Die Kapseln enthalten 25 mg / 100 mg Ciclosporin A aufgelöst in einer Mischung von Öl, Ethanol und Emulgator. In diesem Fall wird die ölige Zubereitung im Magen durch peristaltische Bewegungen dispergiert. Es handelt sich auch hier um ein ineffizientes Verfahren der Öldispergierung.

Alternativ wurde die Ciclosporin A-beladene Ölphase in weiteren Untersuchungen auch schon mit Hilfe der Hochdruckhomogenisation behandelt. Dies führte zu einer feineren O/W Emulsion [Dietl, H., Pharmaceutical preparation containing cyclosporin(s) for oral administration and process for producing same, United States Patent 5,637,317, 1997]. Dieses Patent beinhaltet jedoch keinerlei Daten über die physikalische Stabilität der homogenisierten Emulsion während der Lagerung und keine in vivo Daten, die beweisen, daß der Homogenisationsprozeß zu einer Erhöhung der Bioverfügbarkeit führen kann. Es ist bekannt, daß Ciclosporin - dispergiert in der Ölphase einer O/W Emulsion - nach einigen Tagen präzipitiert und große Kristalle durch auskristallisierenden Arzneistoff in der Emulsion bildet bzw. aus der Ölphase herausgedrängtes Ciclosporin aufschwimmt und auf der Oberfläche einen Rand oder Film bildet. Dieses Problem ist beispielsweise auch für die Sandimmun® Trinkemulsion bekannt. Zusätzlich ist bekannt, daß die Einarbeitung eines Arzneistoffes in die Ölphase einer O/W Emulsion die physikalische Stabilität der Emulsion durch auftretende Koaleszenzneigung verringern kann [S.S. Davis, Pharmaceutical aspects of i.v. fat emulsions, J. Hosp. Pharm., 32, 149-170, 1974]. Die geringe Tröpfchengröße ist nicht der alleinig entscheidende Faktor zur Erhöhung der Ciclosporin Bioverfügbarkeit. Die Homogenisation der Emulsion wird nicht alleine automatisch zur Bioverfügbarkeitserhöhung führen, da die gewöhnliche Ciclosporin A-Resorption zum großen Teil auch von der Gallensalzsekretion beeinflußt wird. [A. Meinzer, E. Müller, J. Vonderscher, Perorale Mikroemulsionsformulierung - Sandimmun Optoral™ / Neoral™, in Pharmazeutische Technologie: Moderne Arzneiformen, R.H. Müller und G.E. Hildebrand (Hrsg.), Wissenschaftliche Verlagsgesellschaft Stuttgart, 1998). Neben dem Ausmaß der Ausschüttung von Gallensalzen ist die Aufnahme von Nahrung während der Arzneistoffresorption ein bedeutender Faktor, der die Bioverfügbarkeit von Ciclosporin beeinflussen kann. Außerdem ist die Arzneistofffreisetzung aus Emulsionen abhängig vom Verteilungskoeffizienten. Dieser Einfluß kann kaum kontrolliert werden, um nicht-variable verlängerte Blutspiegel zu erreichen. Diese Nachteile von O/W Emulsionen (d.h. grobdispersen Emulsionen) sind bereits für andere Ciclosporin Emulsionen beschrieben worden [z.B. A. Tibell et al., Cyclosporin A in fat emulsion carrier. Immunosuppressive effect in vitro, J. Immunolo. 35, 231-236, 1992].

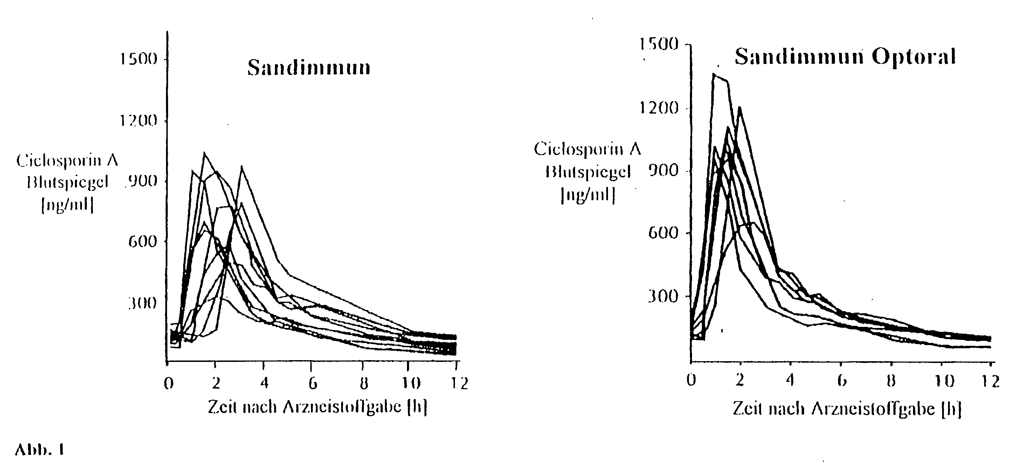

Die nächste Entwicklungsstufe war der Austausch der grobdispersen Sandimmun® Formulierung durch die Mikroemulsion Sandimmun Optoral®. Dies führte zu einer nahezu von der Gallensalzsekretion unabhängigen Resorption von Ciclosporin A [A. Meinzer, E. Müller, J. Vonderscher, Perorale Mikroemulsionsformulierung - Sandimmun Optoral™ / Neoral™, in Pharmazeutische Technologie: Moderne Arzneiformen, R.H. Müller und G.E. Hildebrand (Hrsg.), Wissenschaftliche Verlagsgesellschaft Stuttgart, p. 176, 1998]. Eine Mikroemulsion beinhaltet keine diskreten Tröpfchen. Es handelt sich um eine "kritische Lösung" [B.W. Müller, Mikroemulsionen als neue Wirkstoffträgersysteme, in Pharmazeutische Technologie: Moderne Arzneiformen, R.H. Müller und G.E. Hildebrand (Hrsg.), Wissenschaftliche Verlagsgesellschaft Stuttgart, 161-168, 1998; B.W. Müller, H.J. Franzky, C.J. Kölln, US Patent Nr. 4,719,239, 1988]. Die orale Gabe der Ciclosporin A-Mikroemulsion reduziert die Variabilität der Resorption, führte jedoch zu hohen initialen Blutspiegelpeaks, deutlich über dem Grenzwert von 1000 ng/ml (Abb. 1, rechts). Diese Blutspiegelpeaks sollten in einer optimierten Zubereitung verhindert werden.

Es gibt keine wirksamen topischen Zubereitungen mit Ciclosporin A zur topischen Behandlung (z.B. Psoriasis). In der Literatur wird beschrieben, daß Ciclosporin prinzipiell eine topische Wirkung hat (Clinical Report, Servizio de Medicina, Hospital del Cobre, Rancagua, Chile, Rev. Med. Chil. 1994, Vol. 122; 1404-7). Die Wirksamkeit wurde aber erst nach sechs Monaten Behandlung beobachtet, zusätzlich mußte Dimethylsufoxid als Lösungsmittel in einer Konzentration von 50% eingesetzt werden, was für eine Behandlung wie bei Psoriasis nicht akzeptabel ist. Prinzipielle Therapieeffizienz wurde auch in einem anderen Report berichtet (Intralesional cyclosporine for psoriasis. Relationship of dose, tissue levels and efficacy. J. Gajardo, J. Villaseca, Arch. Dermatol. 1992, Vol. 128; 786-90). Topische Applikation zeigte hier keinen Effekt, was belegt, wie wichtig eine geeignete Arzneiform ist. Hier wurden daher intralaesinale Injektionen vorgenommen, was zur Demonstration der prinzipiellen Effizienz geeignet war, aber für eine Therapie absolut nicht praktikabel ist.

In der Patentschrift US 5 430 021 offenbaren Rudnic et al. eine pharmazeutische Formulierung, die einen Peptidwirkstoff (wie beispielsweise Ciclosporin) umfaßt, der in hydrophobe Partikel aus langkettigen Carbonsäuren oder deren Ester mit langkettigen Alkoholen eingebracht ist. Die hydrophoben Partikel werden mittels Sprühtrocknung aus einer Schmelze, Dispersion oder Lösung gewonnen und weisen eine Partikelgröße zwischen 0.5 und 100 Mikrometer auf.

**Zusammenfassung**: Momentan sind die hauptsächlichen technologischen und biopharmazeutischen Probleme von Ciclosporin A-Formulierungen:
1. die pharmazeutische Qualität und physikalische Stabilität der Zubereitung (z.B. grobdisperse Emulsion, Bildung von Ciclosporin A-Kristallen, Koaleszenz).
2. die hohe Variabilität der Blutspiegel.
3. Blutspiegelpeaks wesentlich > 1500 oder > 1200 oder > 1000 ng/ml, die toxische Nebenwirkungen in verschiedenen Graden begründen.
4. unwirksame topische Formulierungen.

Aufgabe der Erfindung sollte die Beseitigung der oben genannten Probleme bei der Herstellung und hinsichtlich der Wirkung von Ciclosporin-Formulierungen sein. Alternativ kann die Erfindung zur Herstellung von Formulierungen genutzt werden, die den Transport von Ciclosporin in die Dermis zur wirksamen topischen Behandlung ermöglichen.

Diese Aufgabe wird durch einen Arzneistoffträger, der feste Lipidpartikel beladen mit Ciclosporin oder Ciclosporinderivaten natürlichen und/oder synthetischen Ursprungs umfaßt, wobei die Partikel einen Teilchendurchmesser von 10 nm bis 10 µm aufweisen, durch Hochdruckhomogenisation hergestellt worden sind und in einem bei Raumtemperatur festem Zustand vorliegen, wobei die Partikel der Hauptpopulation einen mittleren PCS (mittels Photonenkorrelationsspektroskopie bestimmten) Teilchendurchmesser im Bereich von 40 nm bis 1000 nm besitzen, gelöst.
Die Erfindung stellt damit eine Ciclosporin Formulierung bereit, die eine mittlere Blutspiegelkonzentration im Steady State Bereich von 300 ng/ml bis über 1000 ng/ml, vorzugsweise über 800 ng/ml, insbesondere bis 900 ng/ml, bevorzugt von 400 ng/ml bis 800 ng/ml unter Abwesenheit hoher mittlerer initialer Blutspiegelkonzentrationen wesentlich über 1500 ng/ml, insbesondere über 1200 ng/ml, vorzugsweise über 1000 ng/ml und bevorzugter über 800 ng/ml erzeugt. Die erfindungsgemäße Ciclosporin Formulierung erzeugt eine verlängerte mittlere Blutspiegelkonzentration im Steady State Bereich für einen verlängerten Zeitraum von mindestens 5 h, vorzugsweise mindestens 7 h, insbesondere 9 h.

Das Ausbleiben von Blutspiegelpeaks und die verlängerte Freisetzungszeit wurde durch den Einsatz feiner Partikel von festen Lipiden erreicht. Im Gegensatz zur flüssigen Ölphase einer O/W Emulsion kann aufgrund der festen Lipidmatrix das Freisetzungsprofil durch Diffusion des Arzneistoffs in der sich abbauenden Lipidmatrix gesteuert werden.

Lipide werden aufgrund des lipophilen Charakters des Arzneistoffs Ciclosporin als idealen Kandidaten für die Einarbeitung in Lipidpartikel als Matrixmaterial bevorzugt. Zusätzlich können Lipidpartikel im Gegensatz zu Polymerpartikeln durch Hochdruckhomogenisation im industriellen Maßstab hergestellt werden. [R.H. Müller und S.J. Lucks, Europ. Patent EP 0 605 497 B1, 1996].

Die Herstellung des Arzneistoffträger erfolgt vorzugsweise unter Ausschluß halogenierter organischer Lösungsmittel, und insbesondere bevorzugt unter Ausschluß von organischen Lösungsmitteln überhaupt.

Die zwei grundlegenden Herstellungstechniken sind die Heißhomogenisation und die Kalthomogenisation [C. Schwarz, W. Mehnert, J.S. Lucks, R.H. Müller, Solid lipid nano-particles (SLN) for controlled drug delivery.. I. Production, characterization and sterilization, J. Controlled Rel., 30, 1994, 83-96].

Für die Heißhomogenisationstechnik wird der Arzneistoff in dem geschmolzenen Lipid zunächst gelöst bzw. fein dispergiert. Anschließend wird das arzneistoffbeladene Fett in einer heißen Emulgatorlösung bei Temperaturen über dem Schmelzpunkt des Lipids dispergiert und durch Rühren zu einer Voremulsion verarbeitet. Diese grobe Voremulsion wird anschließend durch Hochdruckhomogenisation bei Drücken zwischen 100 bar und 1 500 bar in einem oder mehreren Homogenisationszyklen fein dispergiert. Die Hochdruckhomogenisation erfolgt ebenfalls bei Temperaturen oberhalb des Schmelzpunktes der Lipidmatrix. Die erhaltene Nanoemulsion wird abgekühlt. Das Fett rekristallisiert und bildet feste Lipidnanopartikel (SLN).

Beim Einsatz der Kalthomogenisationstechnik verbleibt das Fett im festen Zustand, d.h. die Homogenisation findet bei Temperaturen unterhalb des Lipidschmelzpunktes statt. Zuvor wurde das arzneistoffbeinhaltende Fett mit Hilfe einer Mühle, z.B. einer Mörsermühle, zu Mikropartikeln zerkleinert. Die erhaltenen Lipidpartikel werden anschließend in einer kalten Emulgatorlösung dispergiert, und mittels der Hochdruckhomogenisation homogenisiert. Die Scherkräfte und die Kavitation sind hoch genug, um das feste Lipid zu zerkleinern und ultrafeine feste Lipidpartikel, d.h. feste Lipidnanopartikel, zu bilden.

Beide Techniken wurden für die Herstellung von Ciclosporin-beladenen Lipidformulierungen in dieser Erfindung herangezogen.

Der erfindungsgemäße Arzneistoffträger umfaßt dabei insbesondere tensidhaltige oder tensidfreie Partikel eines Lipids oder einer Mischung von Lipiden, die in einem Partikelgrößenbereich von 10 nm bis 100 µm liegen und bei Raumtemperatur fest sind, wobei die Partikel der Hautpopulation einen mittleren Teilchendurchmesser von 40 nm bis 100 µm aufweisen und herstellbar sind, indem entweder eine innere Phase (Lipidphase) in einem Dispersionsmedium (Wasser, wäßrige Lösung oder eine mit Wasser mischbare Flüssigkeit) in geschmolzener oder erweichter Form dispergiert wird, oder eine innere Phase (Lipidphase) in einem Dispersionsmedium in einer festen Form dispergiert wird, wobei die feste Phase hierbei vor dem Dispergierprozeß fein zerkleinert wird.

Die bei Raumtemperatur festem Zustand vorliegen Partikel weisen einen Teilchendurchmesser von 10 nm bis 10 µm auf, wenn sie durch Hochdruckhomogenisation hergestellt worden sind, wobei die Partikel der Hauptpopulation einen mittleren PCS Teilchendurchmesser im Bereich von 40 nm bis 1000 nm besitzen. Bevorzugt weisen die Partikel der Hauptpopulation einen mittleren Teilchendurchmesser im Bereich zwischen 100 nm und 500 nm auf, wobei mit geeigneten ausgewählten Prozeßparametern und Zusätzen die PCS Teilchendurchmesser im Bereich zwischen 40 nm und 100 nm liegen können. Die Hauptpopulation besteht dabei aus der Mehrzahl der Teilchen der Population.

Als weitere Zerkleinerungsvefahren zur Herstellung des erfindungsgemäßen Arzneistoffträgers eignen sich auch Hochgeschwindigkeits-Rührung, Ultraschall-Beschallung oder ein Mahlprozeß, insbesondere mit Einsatz von Flüssigstrahl-(jet stream) oder Luftstrahlmühlen, wobei die festen Partikel der Hauptpopulation einen mittleren Teilchendurchmesser von 0,5 µm bis 100 µm (detektiert mittels Laserdiffraktometrie) aufweisen.

Um das oral zu applizierende Volumen der Endformulierung genügend klein zu halten, ist es bevorzugt, die Lipidmatrix mit 20% des Arzneistoffs Ciclosporin A zu beladen. In früheren Untersuchungen wurde berichtet, daß eine hohe Arzneistoffbeladung der Lipidmatrix, beispielsweise eine 20% Tetracain-Beladung, zu sehr groben Dispersionen führte. Die Partikelaggregation wurde begünstigt und innerhalb der ersten Stunden nach Herstellung trat ein Geliervorgang ein [A. zur Mühlen, C. Schwarz, W. Mehnert, Solid lipid nanoparticles (SLN) for controlled drug delivery - drug release and release mechanism, Eur. J. Pharm. Biopharm., in press, 1998]. Deswegen wurde erwartet, daß die Herstellung einer Ciclosporin Lipidpartikel Dispersion bei vergleichbar hoher Arzneistoffbeladung der Lipidmatrix im Nanometerbereich mit hoher Dispersität (= geringe Partikelgröße) und einer ausreichenden physikalischen Stabilität nicht sehr wahrscheinlich wäre. Jedoch konnte der gegenteilige Effekt beobachtet werden. Die Partikelgröße der Ciclosporin-beladenen Lipid Dispersion nahm mit ansteigendem Ciclosporin Gehalt der Lipidmatrix ab und gleichzeitig verringerte sich die Polydispersität der Dispersion. Ciclosporin begünstigt die Ausbildung ultrafeiner Lipidpartikel von geringer Partikelgröße und hoher Gleichförmigkeit. Die Zugabe von Ciclosporin in die Lipidmatrix erhöht die pharmazeutische Qualität der Lipidnanopartikel Dispersion mit einem Stabilitätsoptimum bei einer Arzneistoffbeladung von 20% (V/V).

Der erfindungsgemäße Arzneistoffträger weist daher einen Gehalt der inneren Phase (Lipidphase) bezogen auf die Gesamtformulierung im Bereich von 0,1% bis 40% (m/m) und insbesondere im Bereich von 1% bis 20% (m/m) auf.

Arzneistoffbeladene Lipidnanopartikel wurden bisher generell als wenig physikalisch stabil beschrieben [C. Schwarz, Feste Lipidnanopartikel: Herstellung, Charakterisierung Arzneistoffinkorporation und -freisetzung, Sterilisation und Lyophilisation, Dissertationsschrift, Freie Universität Berlin, 1995]. Die De-Stabilisation dieser Formulierung erfolgt in drei Stufen:
1. Die Lipidpartikel aggregieren, wie eindeutig durch das Anwachsen der mittleren Teilchendurchmesser erkennbar ist.
2. Die Viskosität der Dispersion steigt stark an und deutet auf einen voranschreitenden Kontakt zwischen den Aggregaten hin.
3. Die Lipidnanopartikel bilden zunächst eine leicht cremige Konsistenz. Später bilden sie feste Gele.

Das Gel ist zusammengesetzt aus einem Netzwerk der Lipidpartikel. Es wurde beobachtet, daß die Bildung von Gelen von einer Reduktion der Fraktion an α-Modifikation und einem gleichzeitigen Anwachsen der Fraktion der β- bzw. β' -Modifikation begleitet war [C. Freitas, R.H. Müller, Long-term stability of solid lipid nanoparticles (SLN™). II. Influence of crystallinity of the lipid and shear forces, submitted to Eur. J. Pharm. Biopharm. 1997].

Lipidpartikel Dispersionen, die physikalisch stabil waren (keine Aggregatbildung, keine Bildung von Gelen), zeigten kaum eine Abnahme bzw. sogar noch eine leichte Zunahme der Fraktion der α-Modifikation während der Lagerung. In diesem Fall wurde kein Lipidanteil in die β- bzw. β' -Modifikation transformiert [C. Freitas, R.H. Müller, Long-term stability of solid lipid nanoparticles (SLN™). II. Influence of crystallinity of the lipid and shear forces, submitted to Eur. J. Pharm. Biopharm. 1997]. Es ist bekannt, daß die Anwesenheit von Arzneistoffen in der Lipidmatrix die Kristallisation in der stabileren β- bzw. β' -Modifikation begünstigt [B. Siekmann, Untersuchungen zur Herstellung und zum Rekristallisationsverhalten schmelzemulgierter i.v. applizierbarer Glyceridnanopartikel, Dissertationsschrift, Techn. Universität Carolo-Wilhelmina zu Braunschweig, 1994].

Partikel aus reinem Lipid, welche normalerweise nach Produktion in einem flüssigen Zustand verblieben und eine verzögerte Rekristallisation nach Tagen oder Wochen zeigten, konnten in ihrer Rekristallisation durch die Einarbeitung von Arzneistoffen beschleunigt werden. Daher wurde erwartet, daß die Ciclosporin-Einarbeitung in die Lipidmatrix die Lipidpartikel Dispersion de-stabilisieren würde. Überraschenderweise trat das Gegenteil ein. Die Bildung der β- bzw. β' -Modifikation wurde durch Ciclosporin A verhindert, die Fraktion der α-Modifikation verblieb unverändert oder wuchs während der Lagerung sogar geringfügig an. Die Ciclosporin A-beladenen Partikel waren physikalisch stabiler als die arzneistofffreien Lipidpartikel, erkennbar an der geringeren Partikelgröße und dem geringeren Partikelwachstum während der Lagerung. Ciclosporin stellt einen Stabilisator für die Lipidpartikel dar und führt zu einer erhöhten physikalischen Stabilität der Lipiddispersion.

Der Einsatz eines Lipids als Arzneistoffträgermatrix hat große Vorteile unter Berücksichtigung der toxikologischen Aspekte. Die meisten Bestandteile von Lipidnanopartikeln besitzen den GRAS-Status oder sind als GRAS-Substanz akzeptiert (GRAS = generally regarded as safe) [Food Additives - GRAS substances, Food Drug Cosmetic Law Reports, Chicago, 1994]. Generell können alle Lipide und alle Emulgatoren, die für die orale Gabe zugelassen sind (z.B. in Tabletten, Kapseln, Pellets, Trinklösungen und Suspensionen) für die Herstellung eingesetzt werden. Typische Lipidmatrix-Materialien sind Glyceride der Fettsäuren, die in Lebensmitteln oder im Körper vorhanden sind. Emulgatoren sind beispielsweise Lecithine, Natriumcholat, Polysorbate wie Polysorbat 80 und Block-Copolymere, wie z.B. Poloxamer 188 (ein Polyethylenpolypropylenoxid A-B-A-Blockcopolymer mit einem mittleren relativen Molekulargewicht von 8350 g/mol, wobei das mittlere relative Molekulargewicht des Polyoxypropylenanteils 1750 g/mol beträgt und der Polyoxyethylenanteil 80% ist). Die genannten Emulgatoren sind sogar für die intravenöse Anwendung zugelassen.

Zusammenfassend kann gesagt werden: In dieser Erfindung wurde Arzneimittelträger bzw. ein Arzneimittel für ein optimales therapeutisches Behandlungsschema mit optimierten Blutspiegeln entwickelt und durch den Einsatz von Ciclosporin A-beladenen ultrafeinen Lipidpartikeln realisiert. Ciclosporin erhöhte die physikalische Qualität der Partikeldispersion durch die Bildung besonders feiner Partikel mit hoher Gleichförmigkeit. Zusätzlich wuchs die physikalische Stabilität nach Inkorporation von Ciclosporin in die Lipidpartikel während der Lagerung der Partikeldispersion an.

| **Kurze Beschreibung der Abbildungen:** | |
|---|---|
| Abb. 1 | Ciclosporin A Blutspiegel nach oraler Gabe von Sandimmun® (links) und von Sandimmun Neoral® (rechts) an Nierentransplantations-Patienten. |
| | |
| Abb. 2 | Ciclosporin A Blutspiegel nach oraler Gabe an Schweinen (Mittelwert von n=3, Dosis 16 mg pro kg). Oben: Sandimmun Neoral® als Referenz, unten: Ciclosporin beladene feste Lipidpartikel (mittlerer PCS Teilchendurchmesser: 157 nm). |
| | |
| Abb. 3 | Partikelgrößenverteilung von arzneistofffreien SLN (links) und Ciclosporin A-beladenen SLN (rechts) erhalten nach 1, 3 und 5 Homogenisationszyklen bei 85 °C (SLN Zubereitung: 10% Imwitor 900 (eine Mischung von Mono-, Di- und Triglyceriden der Palmitin- und Stearinsäure, Monoglyceridanteil ca. 40%), 2,5% Tagat® S (Polyoxyethylenglycerolmonostearat), 0,5% Natriumcholat, 87% Wasser; im Fall einer arzneistoffbeladenen SLN Suspension: 8% Imwitor 900 und 2% Ciclosporin A, 2,5% Tagat® S, 0,5% Natriumcholat, 87% Wasser) (y-Achse: Häufigkeit, x-Achse: µm; Laserdiffraktometrie-Daten). |
| | |
| Abb. 4a | Partikelgrößenverteilung von SLN Dispersionen mit steigendem Gehalt an Ciclosporin A, hergestellt mittels der Heißhomogenisationstechnik bei 85 °C (5%, 10%, 15% und 20% Ciclosporin-Beladung bezogen auf die Lipidmatrix, Formulierungen von Tabelle 2, Laserdiffraktometrie-Daten). |
| | |
| Abb. 4b | Partikelgrößenverteilung von SLN Dispersionen mit steigendem Gehalt an Ciclosporin A hergestellt mittels der Kalthomogenisationstechnik bei 55 °C (5%, 10%, 15% und 20% Ciclosporin-Beladung bezogen |
| | auf die Lipidmatrix, Formulierungen von Tabelle 2, Laserdiffraktometrie Daten). |
| | |
| Abb. 5 | Anwachsen der Partikelgröße (Laserdiffraktometrie Durchmesser d90%) und Anwachsen der Schmelzenthalpie am Beispiel von zwei zum Gelieren neigenden Modellrezepturen (10% Lipid und 5% Lipid) als eine Funktion der Lagerzeit und Lagerungsbedingungen (unter. Streßbedingungen, schütteln bei 40 °C) [nachr-C. Freitas, R.H. Müller, Long-term stability of solid lipid nanoparticles (SLN™). II. Influence of crystallinity of the lipid and shear forces, submitted to Eur. J. Pharm. Biopharm. 1997]. Die Partikelgrößenbestimmung wurde durchgeführt mit Hilfe der Laserdiffraktometrie (Mastersizer E, Malvern Instruments, England), die DSC Analyse erfolgte durch eine Mettler-Toledo DSC 821e/700, Gießen, Deutschland. Die Größenanalyse der 10%-igen Lipidpartikel Suspensionen erfolgte nur an den Tagen 0, 1 und 3 nach Produktion. Am Tag 5 trat eine Gelierung der 10% Formulierung ein, die zu einer cremigen Konsistenz führte, eine Partikelgrößenanalytik war nicht mehr durchführbar. |
| | |
| Abb. 6 | DSC Aufheizkurven von Imwitor 900 Bulk-Material und Imwitor 900 Lipidmatrices mit steigender gelöster Arzneistoffkonzentration von Ciclosporin A von 5% bis 30%. Die DSC Aufheizkurven wurden nach Lösen des Arzneistoffs in der Imwitor 900 Matrix bei 85° für 15 min und Auskristallisieren der Mischung für eine Stunde bei Raumtemperatur gemessen. Die Heizrate beträgt 5 Kelvin pro min, eine Mettler-Toledo DSC 821E/700 Anlage wurde eingesetzt (Mettler-Toledo, Gießen, Deutschland). |
| | |
| | |
| Abb. 7 | Oben: Röntgendiffraktogramme von Ciclosporin A und Imwitor 900 Bulk-Material und von Ciclosporin Abeladenen festen Lipidpartikeln am Tag 1 nach Herstellung (2% Ciclosporin A, 8% Imwitor 900 (d.h. 20% Arzneistoffbeladung bezogen auf die Lipidmatrix), 2,5% Tagat® S, 0,5% Natriumcholat, 87% Wasser). |
| | Unten: Röntgendiffraktogramme von Ciclosporin Abeladenen festen Lipidpartikeln während der Lagerung (bei 25 °C am Tag 1, Tag 7, Tag 14 und Tag 30 nach Herstellung (Nonius PDS120 Pulver-Diffraktometer in einfacher Transmission mit Cu_{Kα}-Strahlung)). |
| | |
| Abb. 8 | Röntgendiffraktogramme von 2% Ciclosporin A-beladenen festen Lipidnanopartikeln (Beispiel 7) am Tag 1 nach der Herstellung (= wäßrige Suspension), direkt nach Lyophilisation und nach 180 Tagen Lagerung der gefriergetrockneten Zubereitung bei 25 °C (Nonius PDS120 Pulver-Diffraktometer in einfacher Transmission mit Cu_{Kα}-Strahlung). Die Diffraktogramme von Ciclosporin und dem Cryoprotektor Trehalose sind als Referenz beigefügt. |
| | |
| Abb. 9 | Bioverfügbarkeit von Ciclosporin SLN gegenüber Sandimmune Neoral nach einer einzigen oralen Verabreichung an 9 gesunde Probanden. Die Formulierung von Ciclosporin SLN wurde gemäß Beispiel 1 hergestellt. Das Material (3kg) wurde in einem Lab 60 APV Homogenisator unter Verwendung von 200 bar Druck in kontinuierlichem Modus und bei 85 °C für 20 Minuten produziert. Die Formulierung wurde 9 gesunden Probanden als Suspension in einer Dosis von Ciclosporin A equivalent zu Sandimmune Neoral (300mg) verabreicht. Die mittleren Blutspiegel sind gezeigt (oben: Sandimmun Neoral; unten: Ciclosporin SLN Suspension). |

**Beispiele der Endformulierung für die Patienten:** Die flüssige Dispersion der Lipidpartikel kann sprühgetrocknet oder gefriergetrocknet werden. Zur oralen Gabe kann das trockene Pulver in Sachets zur Rekonstitution einer Trinksuspension eingefüllt werden. Das Pulver kann auch in Kapseln eingefüllt oder zur Herstellung von Tabletten genutzt werden. Alternativ kann die wäßrige SLN Dispersion nach Einengung der Wasserphase für die Extrusion und Produktion von Pellets oder in der Granulierung für Tabletten genutzt werden. Die parenterale Applikation ist auch möglich, z.B. die intravenöse Gabe. Der ultrafeine Charakter der festen Lipidnanopartikel verhindert die Kapillarblockade und damit auch das Risiko der Embolie.

Mit der Hilfe eines solchen Arzneistoffträgers löst die vorgestellte Erfindung die vorhandenen Probleme durch ein kontrolliertes Design optimierter Blutspiegel mittels Einsatz einer feinen Dispersion physikalisch stabiler fester Lipidnanopartikel.

Ein spezielles therapeutisches Behandlungsdesign wurde entwickelt, um toxische Nebenwirkungen des Ciclosporins zu minimieren und die therapeutische Effizienz zu erhöhen. Eine Erhöhung der therapeutischen Effizienz erfolgte durch Erniedrigung der Variabilität der Absorption und durch die Erzielung einer verzögerten Freisetzung mit länger anhaltenden Blutspiegelwerten (steady state) im optimalen Therapiebereich. Der optimierte mittlere Blutspiegel dieser Erfindung verhindert Blutspiegelpeaks wesentlich > 1500 ng/ml, insbesondere wesentlich > 1200 ng/ml, bevorzugter > 1200 ng/ml, vorzugsweise > 1000 ng/ml und idealerweise > 800 ng/ml. Der Blutspiegel verbleibt für eine gewisse Zeitperiode in dem Bereich zwischen 300 ng/ml und 900 ng/ml, vorzugsweise im Bereich zwischen 400 ng/ml und 800 ng/ml. Der Zeitbereich der gleichbleibenden Ciclosporin A-Blutspiegel beträgt mindestens 5 h, vorzugsweise 7 h und im Idealfall länger als 8 h mit einer Arzneimittelkonzentration von 300 ng/ml bis 900 ng/ml im Blut. Die Erfindung ermöglicht die Herstellung von topischen Ciclosporin A Formulierungen, die den Transport von Ciclosporin A in die Dermis fördern.

Abb. 1 (links) zeigt die Variabilität der Blutspiegel nach oraler Gabe von Sandimmun® bei Patienten nach Nierentransplantation. [nach: A. Meinzer, E. Müller, J. Vonderscher, Perorale Mikroemulsionsformulierung - Sandimmun Optoral™ /Neoral™, in Pharmazeutische Technologie: Moderne Arzneiformen, R.H. Müller und G.E. Hildebrand (Hrsg.), Wissenschaftliche Verlagsgesellschaft Stuttgart, p. 175, 1998].

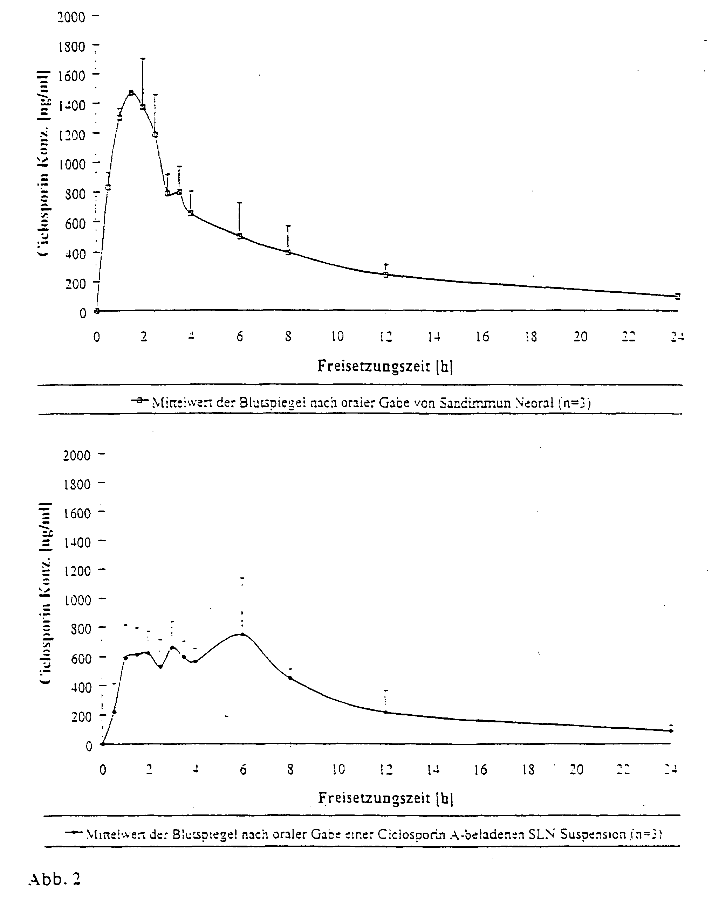

Abb. 2 (unten) zeigt als Vergleich das Ergebnis der Verwendung des erfindungsgemäßen Arzneimittelträges bzw. des erfindungsgemäßen Behandlungsdesigns den mittleren Blutspiegel einer Schweinestudie, erhalten von drei Schweinen (Beispiel 1).

Der mittlere Blutspiegel dieses erfindungsgemäßen Behandlungsdesigns zeigt keinen Peak über 800 ng/ml über eine Zeitperiode von etwa 9 h bei einem Blutspiegel im Bereich von 300 ng/ml bis 900 ng/ml.

**Biopharmazeutische Eigenschaften:** Die perorale Gabe des Ciclosporin-beladenen Arzneimitelträgers dieser Erfindung führt zu verlängerten Blutspiegeln mit geringer Variabilität und dem Ausbleiben hoher toxischer Blutspiegelpeaks und somit dem Ausbleiben oder der Minimierung von Arzneimittelnebenwirkungen (wie z.B. die Nephrotoxizität von Ciclosporin).

**Qualität der Lipidpartikel:** Feste Lipidpartikel wurden durch Dispergierung einer Ciclosporin-beladenen Lipidmatrix in einer Emulgatorlösung hergestellt. Die Einarbeitung von Ciclosporin in die Lipidmatrix verbessert die Dispersität des Lipids und erhöht die Feinheit und Größengleichheit der Lipidpartikel nach Dispergierung, verglichen zu arzneistofffreien Lipidpartikeln, hergestellt unter gleichen Bedingungen. Die Einschlußrate des Arzneistoffs in den Lipidpartikeln kann durch Steigerung des Ciclosporin Gehaltes in der Formulierung erhöht werden.

**Physikalische Stabilität**: Es konnte gezeigt werden, daß eine Partikelaggregation und Gelbildung (= Destabilisierung) bei Lipiddispersionen einhergeht mit der Umlagerung des Lipids von der α-Modifikation in die β- bzw. β' -Modifikation. Die Einarbeitung von Ciclosporin stabilisiert die Bildung der α-Modifikation, verhindert so die Umwandlung in die β- bzw. β' -Modifikation bei Lagerung und zeigt eine erhöhte physikalische Stabilität der Partikeldispersion.

Die Erfindung wurde verglichen mit dem gegenwärtig aktuellen Produkt Sandimmun Neoral®. Abb. 2 (oben) zeigt die Blutspiegelkurve nach oraler Applikation einer identischen Dosis. Der mittlere Blutspiegel von Sandimmun Neoral® zeigt einen initialen Blutspiegelpeak von etwa 1.500 ng/ml und einen Blutspiegel im Bereich von 300 ng/ml bis 900 ng/ml lediglich über einen Zeitraum von 6 h. Im direkten Vergleich zeigen beide Formulierungen, die Mikroemulsion und die Erfindungsformulierung (Abb. 2), geringe Standardabweichungen und eine ähnliche Variabilität der Bioverfügbarkeit an. Die Erfindungsformulierung vermeidet jedoch die hohe Variabilität der Blutspiegel des bisher bekannten Sandimmun® (Abb. 1, links) sowie die hohen Blutspiegelpeaks von Sandimmun Optoral® (Abb. 1, rechts und Abb. 2, oben) und erzeugt länger anhaltende Blutspiegel (steady state) mit geringer Variabilität.

### Detaillierte Beschreibung der Erfindung und der verwendeten Substanzen

Die Erfindung ermöglicht ein optimales therapeutisches Behandlungsschema, dessen optimale Blutspiegel durch den neuartigen Arzneimittelträger, basierend auf festen, Ciclosporin-beladenen Lipidpartikeln, erreicht wird. Die Lipidpartikel sind für die orale und/oder parenterale Applikation entwickelt worden. Die Lipidmatrix kann ein Ciclosporin oder eine Mischung von zwei oder mehreren Ciclosporinen natürlicher oder synthetischer Herkunft beinhalten. Die Lipidpartikel werden durch Hochdruckhomogenisation hergestellt. Um feine Partikel vorzugsweise im Nanometerbereich zu erreichen, kann beispielsweise die Homogenisation mit einem Kolbenspalthomogenisator, wie bereits in R.H. Müller und S.J. Lucks, Europäisches Patent EP 0 605 497 B1, 1996 beschrieben, eingesetzt werden. Alternativ ist die Homogenisation mit Hilfe eines Mikrofluidizers möglich. Um Lipidpartikel im Bereich von Nanometern bis zu einigen Mikrometern (100 nm bis zu 10 µm) zu erhalten, kann die Dispergierung der Lipidpartikel auch bei einer niedrigeren Leistungsdichte, beispielsweise durch Ultrabeschallung oder mit Hochgeschwindigkeitsrührern erfolgen. Alternativ kann die Dispergierung mittels einer Luftstrahlmühle oder einer Flüssigmahlung durch eine Kolloidmühle durchgeführt werden.

Ölige Dispersionen, wie Sandimmun®, zeigen eine Variabilität der Bioverfügbarkeit zwischen 10% und 60% [T. Beveridge, A. Gratwohl, F. Michot, W. Niederberger, E. Nüesch, K. Nussbaumer, P. Schaub, and B. Speck, Cyclosporin A: pharmacokinetics after a single dose in man and serum levels after multiple dosing in recipients of allogenic bone-marrow grafts, Curr. Ther. Res. 30, 1981, 5-18]. Die Gabe einer Mikroemulsion als kritische Lösung von Ciclosporin (Sandimmun Optoral®) reduzierte die Variabilität der Bioverfügbarkeit, führte jedoch zu noch höheren Blutspiegelpeaks nach der oralen Gabe als bei Sandimmun® (Abb. 1, rechts). Diese hohen Blutspiegelpeaks sind verantwortlich für die toxischen Nebenwirkungen von Ciclosporin A, die während der Behandlung der Patienten auftreten können, z.B. der Nephrotoxizität [Martindale, 29^{th} edition, J.E.F. Reynolds (edt.), London, The Pharmaceutical Press, 1989].

Ciclosporin-beladene Lipidpartikel sind lipidhaltige Dispersionen und keine_Lösungen. Deswegen wurde erwartet, daß die Ciclosporin-beladenen festen Lipidpartikel ebenso wie die bekannte lipidhaltige Sandimmun® Zubereitung die bekannte Variabilität der Bioverfügbarkeit zeigen und daß die Reproduzierbarkeit der Absorption nicht ansteigt. Überraschenderweise zeigte die Ciclosporin-beladene Lipidpartikel (SLN) Suspension eine Reproduzierbarkeit der Bioverfügbarkeit ähnlich der der Sandimmun Neoral® Zubereitung, jedoch ohne die toxischen Blutspiegelpeaks (Abb. 2). Die SLN-Formulierung zeigte sogar eine verlängerte Freisetzung des Arzneistoffs und daraus sich ergebend verlängerte Blutspiegel im Bereich von 400 ng/ml bis 800 ng/ml. Solche konstanten Blutspiegel unter Abwesenheit der Blutspiegelpeaks sind das optimale Behandlungsdesign, das erfindungsgemäß möglich gemacht wird. Blutspiegel von Ciclosporinen > 1000 ng/ml, insbesondere > 1200 ng/ml, speziell wesentlich > 1500 ng/ml werden aufgrund ihrer toxischen Nebeneffekte als kritisch angesehen. Idealerweise sollte der Blutspiegel bevorzugt in dem therapeutischen Bereich zwischen 400 ng/ml bis 800 ng/ml liegen.

Parameter zum Nachweis der Qualität und toxikologischen Akzeptanz von Ciclosporin Formulierungen sind deswegen die Prozentanteile der AUC (area under the curve) des Blutspiegels über 800 ng/ml (AUC_{>800ng/ml}) und über 1000 ng/ml (AUC_{>1000ng/ml}). Konsequenterweise ist die Maximalkonzentration Cₘₐₓ auch ein wichtiger Parameter, der die Konzentration von 1000 ng/ml nicht überschreiten sollte. Ein hoher initialer Blutspiegelpeak ist nicht wünschenswert, d.h. die Zeit bis zum Erreichen von Cₘₐₓ (und auch Tₘₐₓ) sollte nicht so kurz sein, sondern eher zu späteren Zeiten detektiert werden. Aufgrund der verzögerten und gleichmäßigen Freisetzung aus Ciclosporin-beladenen Lipidpartikeln ist das Erreichen von Tₘₐₓ aus Lipidpartikeln im Gegensatz zur Sandimmun Neoral® Referenzzubereitung erst zu einem späteren Zeitpunkt detektierbar.

Tab. 1 zeigt, daß die erfindungsgemäße Ciclosporin SLN-Formulierung diese Erfordernisse erfüllt und belegt die durch die Erfindung erzielte Verbesserung der Therapie.

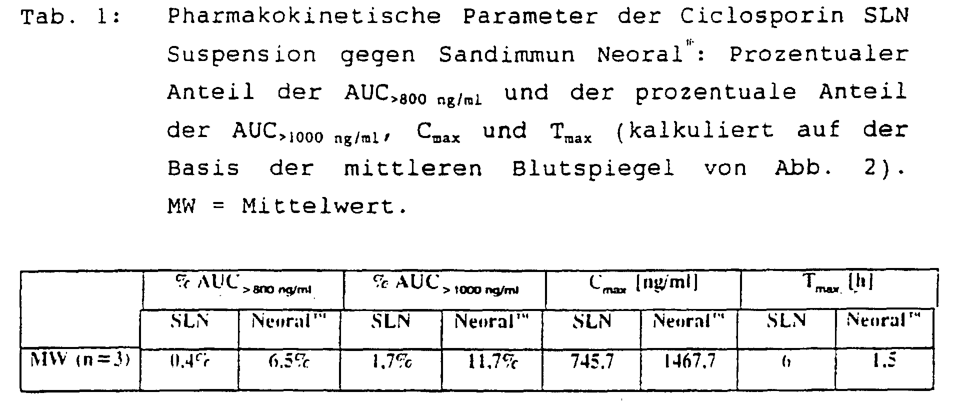

Normalerweise ist die Qualität von Lipidpartikeln (Feinheit und Gleichförmigkeit der Partikelgröße) am höchsten, wenn die Arzneistoffbeladung niedrig bleibt [A. zur Mühlen, C. Schwarz, W. Mehnert, Solid lipid nanoparticles (SLN) for controlled drug delivery - drug release and release mechanism, Eur. J. Pharm. Biopharm., accepted, 1997]. Die Erhöhung der Arzneistoffkonzentration führte bei den Modellarzneistoffen Tetracain und Etomidat zur Partikelaggregation und schließlich bei einer 10%igen Etomidatkonzentration zur Bildung eines Gels [C. Schwarz, Feste Lipidnanopartikel: Herstellung, Charakterisierung Arzneistoffinkorporation und -freisetzung, Sterilisation und Lyophilisation, Dissertationsschrift, Freie Universität Berlin, 1995]. Mit wachsender Ciclosporin-Beladung von 0% bis 20% trat das Gegenteil ein (Tab. 2, Beispiel 2):
1. Die Qualität der Partikel gemessen an der Partikelgröße und -verteilung der arzneistoffbeladenen SLN war besser, als die der arzneistofffreien SLN (d.h. kleinere, feinere Partikel mit enger Verteilung).
2. Eine steigende Arzneistoffbeladung erhöhte die Qualität der Partikel der Suspension (Tab. 3, Beispiel 2).

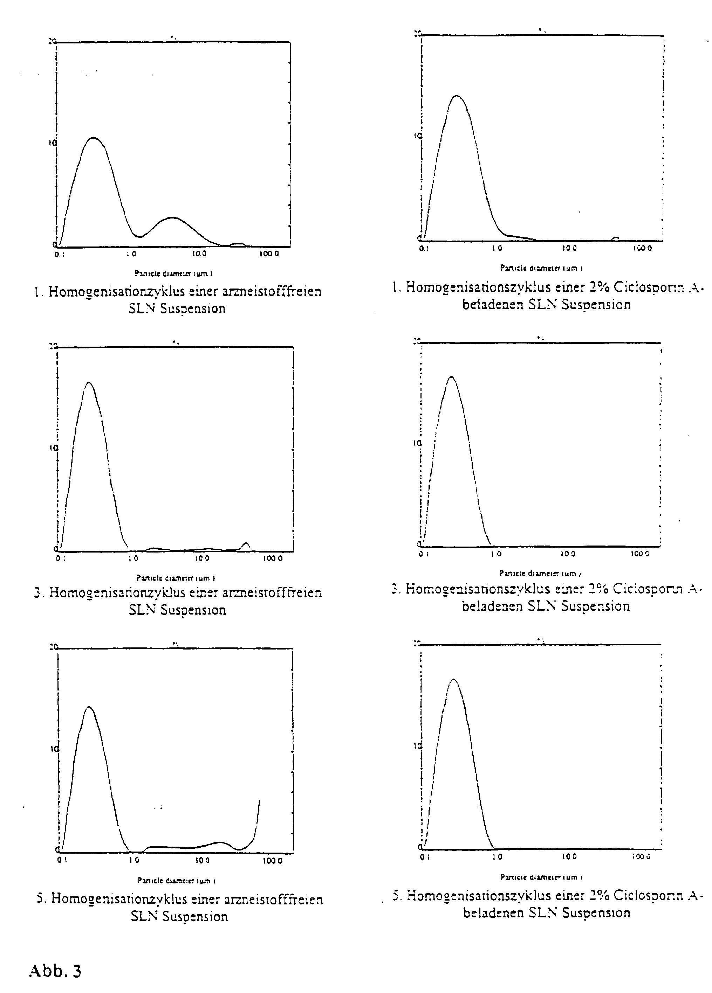

Die Produktion einer arzneistofffreien SLN Suspension führte zur Bildung einer breiten Partikelgrößenverteilung (gemessen mit Hilfe der Laserdiffraktometrie). Mehr als 25% der Partikel waren größer als 1 µm bei einer Größe bis zu 80 µm (Abb. 3, oben links). Drei Homogenisationszyklen reduzierten die Fraktion der Partikel > 1 µm, jedoch nicht die Breite der Verteilung (Abb. 3, Mitte links). Fünf Homogenisationszyklen führten sogar zu einer Partikelaggregation, wie sie durch ein Anwachsen der Verteilungswerte der Partikelgrößenverteilungskurve im Größenbereich von 60 µm bis 80 µm erkennbar ist (Abb. 3, unten links).

Im Gegensatz dazu führte bei einer Ciclosporin-beladenen SLN Zubereitung bereits ein Homogenisationszyklus zu einer sehr engen Partikelgrößenverteilung mit einem geringen Anteil von Partikeln > 1 µm (etwa 5%) nach Beladung der Lipidpartikel mit 20% des Arzneistoffs Ciclosporin (Abb. 3, oben rechts). Drei Homogenisationszyklen führten zu einer extrem gleichförmigen Verteilung mit weniger als einem Prozent der Partikel > 1 µm (Abb. 3, Mitte rechts, Tab. 3 in Beisp. 2). Die bei der arzneistofffreien SLN Suspension beobachtete Destabilisation (Aggregation und Bildung von großen Partikeln) nach fünf Homogenisationszyklen trat bei den Ciclosporin-beladenen Partikeln nicht auf. Die Größenverteilung war praktisch identisch zu der Größenverteilung nach drei Homogenisationszyklen (Abb. 3, Mitte und unten rechts).

Lipidpartikel wurden mit steigender Ciclosporin Konzentration, bezogen auf die Lipidmatrix, hergestellt: 5%, 10%, 15%, 20% (Zubereitung: Beisp. 2, Tab. 2). Die Größenanalysenwerte für Lipidpartikel, die mit der Heißhomogenisationstechnik hergestellt wurden, sind in Tab. 3 (Beisp. 2, Laserdiffraktometrie Daten, Volumenverteilungswerte) gezeigt. Der LD-Durchmesser d50% sank mit steigender Arzneistoffbeladung, bei einer 20%igen Ciclosporin-Beladung bezogen auf die Lipidmatrix betrug der d50% Teilchendurchmesser 310 nm. Eine extreme Abnahme wurde für die volumen-bezogenen Partikeldurchmesser d95% und d99% gemessen. Der Partikeldurchmesser d99% ist ein sehr empfindlicher Parameter, um die Größengleichförmigkeit der Partikelpopulation zu beweisen. Er ist besonders empfindlich, wenn die Kalkulation der Partikelgröße über die Volumenverteilung erfolgt. Die starke Abnahme beweist die Reduktion in der Fraktion von Partikeln im µm-Bereich und auf diese Weise einen Zuwachs der Größengleichförmigkeit. So sank z.B. der Durchmesser d99% von etwa 60 µm (Mittelwert von n=3, 5% Ciclosporin-Beladung der Lipidphase) auf einen Wert von 860 nm (Mittelwert aus n=3, 20% Ciclosporin-Beladung der Lipidmatrix).

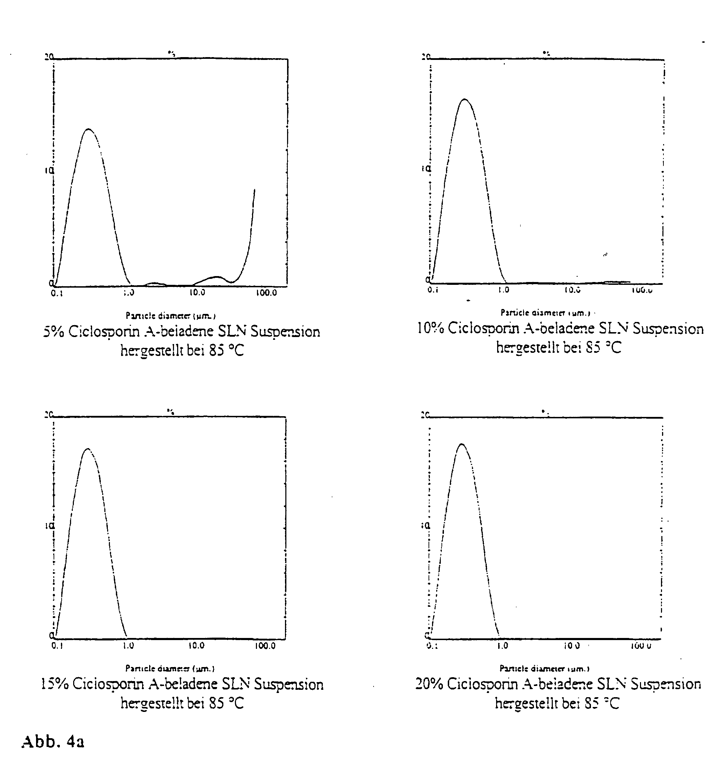

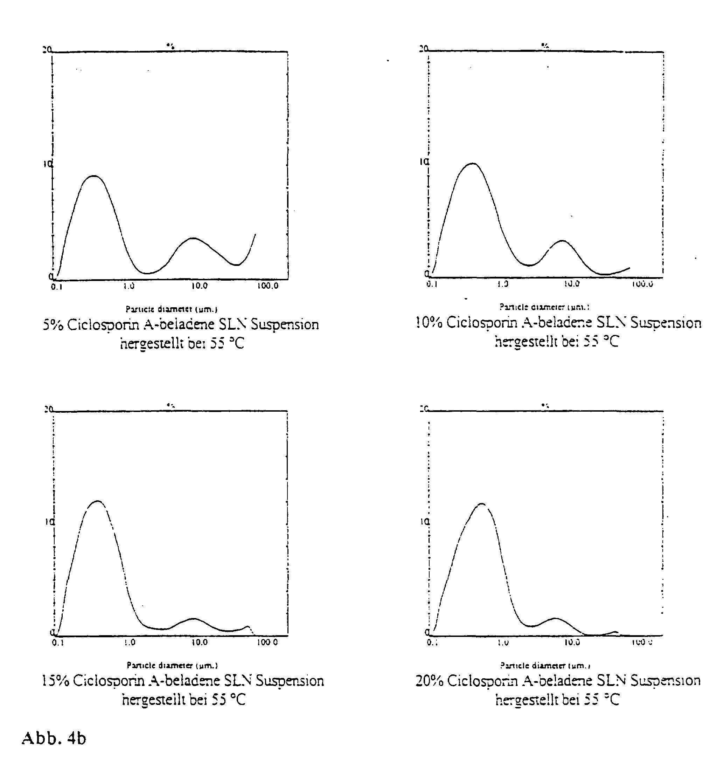

Die Partikelgrößenverteilung der Formulierungen mit ansteigender Ciclosporin Konzentration aus Tab. 3 sind in Abb. 4a (Heißhomogenisation bei 85 °C) und in Abb. 4b (Kalthomogenisation bei 55 °C) graphisch dargestellt. Derselbe Effekt - ansteigende Gleichförmigkeit mit ansteigender Ciclosporin Konzentration innerhalb der Lipidmatrix - wurde bei Anwendung der Kalthomogenisationstechnik beobachtet (Beispiel 3, Tab. 4).

Die Einkapselungsrate des Arzneistoffs in dew Lipidpartikeln wird bestimmt durch die Löslichkeit in der Matrix C₀ (Öl/Lipidphase) und dem Dispersionsmedium C_{W} (Wasser), d.h. aufgrund des Verteilungskoeffizienten k (=C₀/C_{W}). Daher wird bei anwachsender Arzneistoffkonzentration in der Formulierung eine konstante Einkapselungsrate erwartet, wenn man gleichzeitig unterhalb der Maximallöslichkeit des Arzneistoffs in einer der beiden Phasen bleibt, d.h. Lipid oder Wasser. Beispielsweise führt die Übersättigung der Wasserphase aufgrund der erhöhten Temperatur und daraus folgend der erhöhten Löslichkeit des Arzneistoffs zur Bildung von Arzneistoffkristallen nach dem Abkühlen der Dispersion (z.B. Prednisolon in [A. zur Mühlen, C. Schwarz, W. Mehnert, Solid lipid nano-particles (SLN) for controlled drug delivery - drug release and release mechanism, Eur. J. Pharm. Biopharm., in press 1998]). Im Gegensatz dazu wurde mit ansteigender Arzneistoffkonzentration von 0,5% bis 2,0% bezogen auf die Gesamtformulierung (= 5% bis 20% Ciclosporin A kalkuliert als Prozentsatz der Lipidphase) ein Ansteigen der relativen Einkapselungsrate von 95,4% bis zu 97,8% nach der Herstellung mittels der Heißhomogenisationstechnik bei 85 °C erhalten (Beispiel 5, Tab. 5). Nach Herstellung der Lipiddispersion mittels der Kalthomogenisationstechnik wurde ein Anstieg der relativen Einkapselungsrate von 78,5% bis 93,9% erzielt (Beispiel 6, Tab. 6). In der Wasserphase wurden keine Arzneistoffkristalle gefunden. Die Konzentration des Arzneistoffs in der Wasserphase verblieb unterhalb der Sättigungslöslichkeit der emulgatorhaltigen Wasserphase.

Die Einarbeitung von Ciclosporin - besonders bei ansteigenden Konzentrationen - erhöhte die Qualität der produzierten Partikel, insbesondere die Feinheit, Gleichförmigkeit und die Einkapselungsrate des Arzneistoffs in der Lipidmatrix. Zusätzlich wurde die physikalische Stabilität der Arzneistoff-beladenen Formulierung während der Lagerung erhöht. Als physikalische Instabilitäten gelten Partikelaggregation und insbesondere im Fall von Lipidpartikeln die Bildung von Gelen. Arzneistofffreie Imwitor 900 Lipidpartikel zeigten eine hohe Polydispersität nach Herstellung und aggregierten stark während der ersten 5 Tage der Lagerung. Sie bildeten große mikroskopisch erkennbare Aggregate mit einer Größe von etwa 0,5 mm bis 1 mm. Lipidpartikel beladen mit 20% Ciclosporin waren eindeutig stabiler, und dies sogar bei Lagerung unter Streßbedingungen bei 40 °C. Beispielsweise stieg, bezogen auf die Volumenverteilung der Partikel, der LD Durchmesser d50%-lediglich von 0,32 µm auf 0,40 µm, der LD Durchmesser d90% von 0,62 µm auf 0,84 µm an (nach 3-tägiger Lagerung).

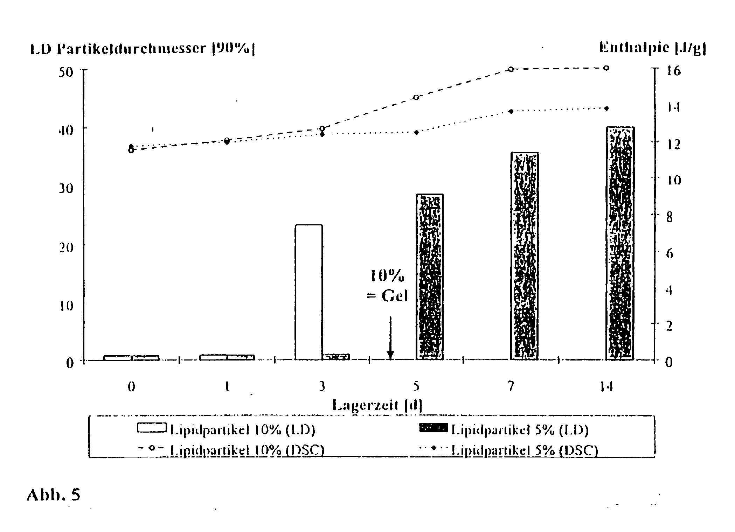

In vorhergehenden Arbeiten mit Lipidpartikeln wurde festgestellt, daß die Lipidpartikel nach der Herstellung hauptsächlich in der β' /βᵢ- bzw. β-Modifikation auskristallisierten und nur teilweise in der weniger stabilen α-Modifikation. Im Falle von physikalisch instabilen Partikeln nahm der Anteil der β' - bzw. βᵢ-Modifikation während der Lagerung zu, der Anteil der α-Modifikation nahm ab bei gleichzeitiger Partikelaggregation und Bildung von Gelen. Zusätzlich kam es zu einem Anwachsen der Schmelzenthalpiewerte während der Partikelaggregation, die Schmelzenthalpiewerte stiegen nochmals bei der Bildung von Gelen [C. Freitas, R.H. Müller, Long-term stability of solid lipid nanoparticles (SLN™). II. Influence of crystallinity of the lipid and shear forces, submitted to Eur. J. Pharm. Biopharm. 1997]. Abb. 5 zeigt das Anwachsen der Partikelgröße (LD-Daten), die Bildung von Gelen und das Anwachsen der Schmelzenthalpie (DSC-Daten) von instabilen Partikeln.

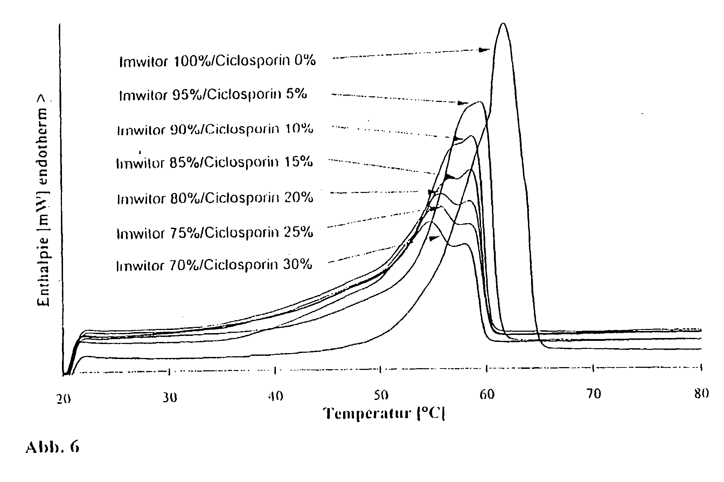

Im Falle physikalisch stabiler Lipidpartikel Dispersionen zeigte der Anteil der β- bzw. β' -Modifikation wenig oder keine Änderung. Der Anteil der α-Modifikation verblieb unverändert oder stieg sogar etwas an. Ein Ansteigen der Schmelzenthalpie war kaum zu beobachten. Ciclosporin fördert die Ausbildung der α-Modifikation bei gleichzeitiger Reduktion der β- bzw. β' -Modifikation. Dieser Effekt wird immer deutlicher bei steigender Konzentration von Ciclosporin in der Lipidmatrix (Beispiel 4, Abb. 6).

Bei Ciclosporin-beladenen Lipidpartikeln kam es nur zu einem geringen Anwachsen der Schmelzenthalpie, z.B. 9,2 J/g am ersten Tag nach der Herstellung, 9,5 J/g am Tag 14 (20% Ciclosporin-Beladung in der Lipidmatrix, Lagerung unter Streßbedingungen bei 40 °C). Diese Eigenschaften der Lipidpartikel sind in dem Zusatz von Ciclosporin begründet und stehen voll in Übereinstimmung mit der beobachteten erhöhten physikalischen Stabilität im Vergleich zu arzneistofffreien Imwitor 900 Lipidpartikeln.

Es ist von Suppositorien bekannt, daß in die Lipidmatrix eingearbeitete Arzneistoffe während der Lagerung aus der Lipidmatrix herausgedrängt werden können (Arzneistoffexklusion) [B.W. Müller, Suppositorien, Wissenschaftliche Verlagsgesellschaft Stuttgart, 1989]. Diese Arzneistoffauslagerung tritt auch beim Verdünnen des öligen Emulsionskonzentrates Sandimmun® in wäßrigen Dispersionsmedien ein und führt zur Ausbildung großer Arzneistoffkristalle in der Wasserphase oder Aufschwimmen des Arzneistoffs auf der Wasseroberfläche. Aus diesem Grund liegt die klassische Sandimmun® Formulierung wasserfrei als öliges Emulsionskonzentrat vor. Das Phänomen der Arzneistoffexklusion ist ein generelles Problem für alle O/W Dispersionen, z.B. auch andere Ciclosporin-beladene Emulsionen [Dietl, H., Pharmaceutical preparation containing cyclosporins(s) for oral administration and process for producing same, United States Patent 5,637,317, 1997]. Der flüssige Zustand der Fettphase in Emulsionen erleichtert die Arzneistoffexklusion verglichen zu festen Suppositorien. Die Arzneistoffexklusion ist offensichtlich weniger ausgeprägt, wenn die Öltropfen sehr fein sind, z.B. im unteren Nanometerbereich wie beispielsweise im Fall der Verdünnung der Mikroemulsion Sandimmun Neoral® in wäßrigen Dispersionsmedien.

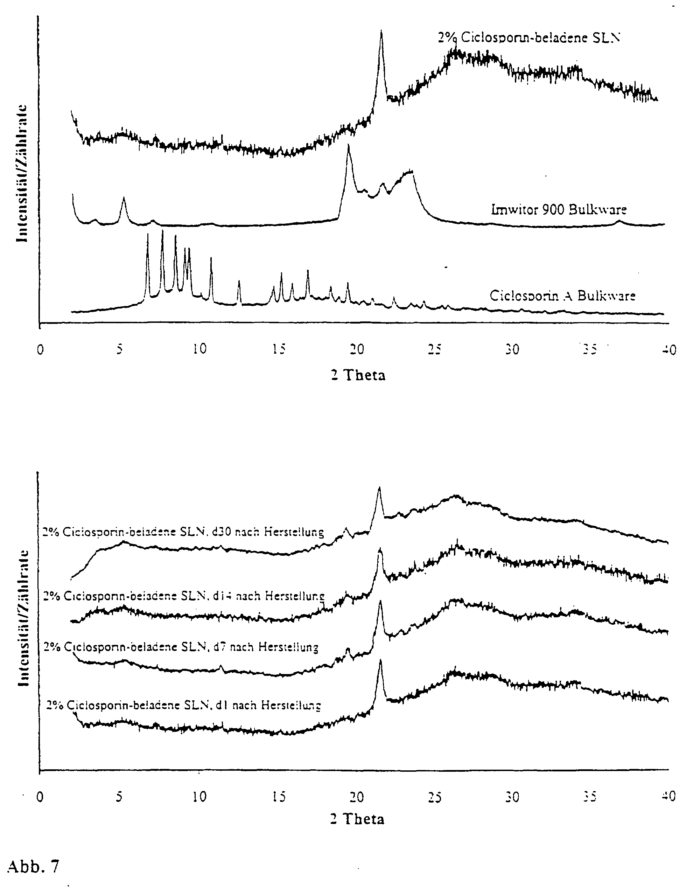

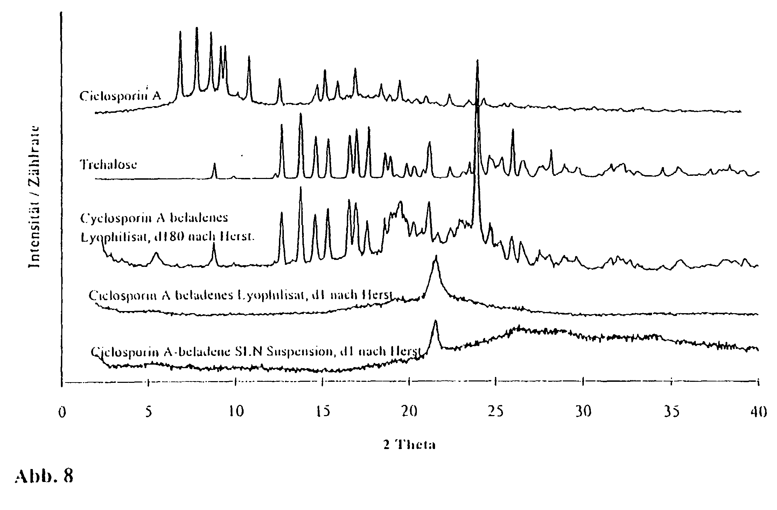

Ausgehend von diesen Tatsachen sind der feste Zustand der Lipidpartikel und die feine Tropfengröße die Hauptvorteile, um die Arzneistoffexklusion zu verhindern oder zumindest stark zu reduzieren. Das Vorliegen des kristallinen Zustands kann durch Röntgendiffraktogramme der wäßrigen festen Lipidpartikel Dispersion untersucht werden. Die Diffraktogramme zeigen während 30-tägiger Lagerung keine Änderung (Abb. 7, unten). Grundsätzlich sind trockene Produkte unter dem Gesichtspunkt der Langzeitstabilität vorzuziehen. Im Gegensatz zu Dispersionen begünstigen sie die Konservierung der physikalischen Stabilität unter Berücksichtigung wirtschaftlicher Aspekte. In den meisten Ländern sind Produkte mit einer langen Haltbarkeit, d.h. einer minimalen physikalischen Stabilität, von 3 Jahren erforderlich. Deswegen wurden die festen Lipidpartikel in trockene Produkte durch Gefriertrocknung (Beispiel 7) und durch Sprühtrocknung (Beispiel 8) überführt. Röntgendiffraktometrie-Untersuchungen der gefriergetrockneten Pulver nach 6-monatiger Lagerung zeigten die Erhaltung der Struktur der Partikel und des amorphen Charakters des in die Lipidmatrix eingearbeiteten Ciclosporins. Es konnten keine Ciclosporin Kristalle detektiert werden, nur kristalline Streupeaks der Trehalose als Cryoprotektor der Formulierung waren nach 180 Tagen Lagerung detektierbar (Abb. 8).

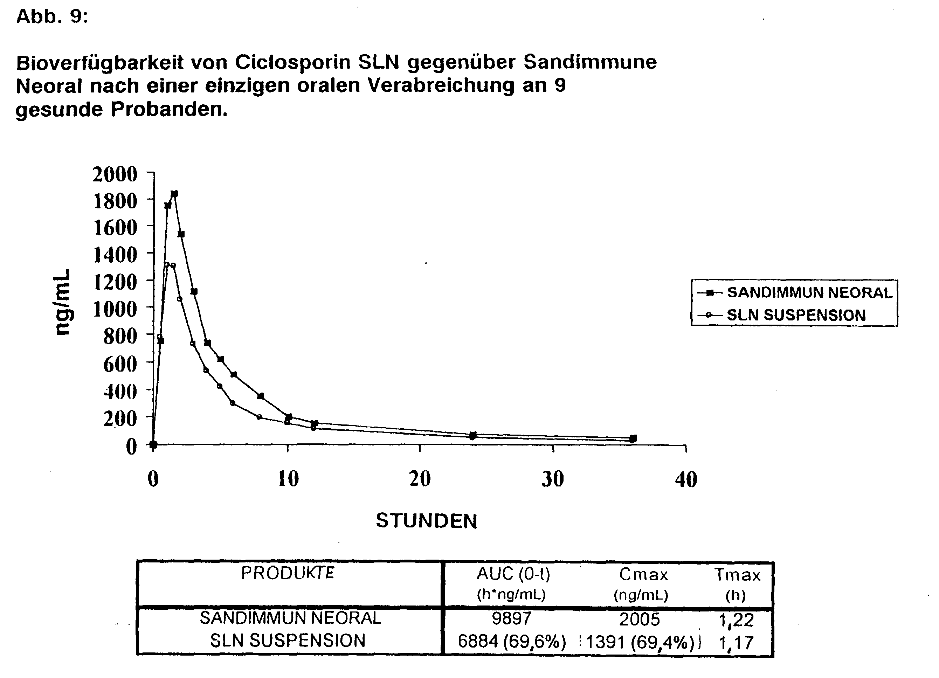

Bioverfügbarkeit: Bioverfügbarkeit von Ciclosporin SLN gegenüber Sandimmune Neoral nach einer einzigen oralen Verabreichung an 9 gesunde Probanden. Die Formulierung von Ciclosporin SLN wurde gemäß Beispiel 1 hergestellt. Das Material (3kg) wurde in einem Lab 60 APV Homogenisator unter Verwendung von 200 bar Druck in kontinuierlichem Modus und bei 85 °C für 20 Minuten produziert. Die Formulierung wurde 9 gesunden Probanden als Suspension in einer Dosis von Ciclosporin A equivalent zu Sandimmune Neoral (300mg) verabreicht. Die mittleren Blutspiegel sind in Abb. 9 gezeigt (oben: Sandimmun Neoral; unten: Ciclosporin SLN Suspension).

Die Erfindung basiert darauf, daß gefunden wurde, daß die Zugabe von Ciclosporin zu einer Lipidmatrix die Dispersität des Lipids erhöht, die Bildung kleiner Partikel begünstigt, die Gleichförmigkeit der Partikelgröße zunimmt, die Einkapselungsrate mit steigender Ciclosporin-Beladung ansteigt, die physikalische Stabilität der Partikeldispersion während der Lagerung erhöht wird, die Bildung der α-Modifikation innerhalb der Lipidmatrix fördert und die Struktur der Lipidmatrix insbesondere die Fraktion der α-Modifikation des Lipids und den amorphen Charakter des eingeschlossenen Arzneistoffs auch nach Lagerung beibehält, insbesondere in Form eines trockenen Partikelproduktes (z.B. erhalten durch Gefriertrocknung). Ein besonderes Merkmal dieser Erfindung ist deswegen der Einsatz von Ciclosporin im Herstellungsprozeß von Lipidpartikeln.

Die Herstellung von Lipidpartikeln gemäß der Erfindung findet durch Dispergierung der Ciclosporin-beladenen Lipidphase in ihrer geschmolzenen Form oder in festem Zustand statt. Unterschiedliche Dispergierungsprozesse können angewendet werden. Die vorzugsweise benutzte Technik ist die Hochdruckhomogenisation, wie sie bereits von Müller und Lucks beschrieben wurde [R.H. Müller and J.S. Lucks, Europäisches Patent EP 0 605 497 B1, 1996]. Die Hochdruckhomogenisation führt zu Partikeln mit einem durch Photonenkorrelationsspektroskopie bestimmten mittleren Teilchendurchmesser im Bereich von etwa 40 nm bis 1000 nm, d.h. zu sogenannten festen Lipidnanopartikeln (SLN® ) beladen mit Ciclosporin. Alternativ zu dieser Herstellungstechnik können auch andere Dispergiertechniken angewendet werden, z.B. Homogenisation mit Hilfe eines Microfluidizers (Microfluidics Inc. USA). Ciclosporin-beladene Partikel mit einer größeren Partikelgröße können durch Dispergierung des geschmolzenen oder festen Fettes mit Hilfe eines Hochgeschwindigkeitsrührers oder anderer Dispergierwerkzeuge hergestellt werden, wie von R.H. Müller bereits in der Applikation PCT/EP97/06893 beschrieben wurde. Es ist daher eine Zubereitung von Matrix-Arzneiformen (z.B. Tabletten, Pellets) basierend auf Ciclosporin-beladenen Mikropartikeln möglich. Diese Dispergiergeräte haben eine niedrigere Leistungsdichte im Bereich der Dispergierzone und führen daher zu größeren Lipidpartikeln, z.B. mit mittleren Teilchendurchmessern von einigen µm bis zu 20 µm oder Partikelpopulation im Bereich von 40 µm bis 100 µm, wie in der Literatur und Standardlehrbüchern bereits ausgiebig beschrieben ist.

Beispielsweise wird Ciclosporin bei erhöhten Temperaturen (wie 80 °C bis 90 °C) in der geschmolzenen Lipidphase gelöst oder dispergiert. Alternativ kann Ciclosporin in das Lipid durch Präzipitation der lipophilen Matrix aus einem Lösungsmittel, in dem Lipid und Ciclosporin gleichzeitig löslich sind, inkorporiert werden. Die Lipidmatrix kann Ciclosporin in molekulardisperser Form, in amorphen Clustern oder in Form ultrafeiner Kristalle (z.B. Ciclosporin-Nanokristalle in Lipidpartikeln) beinhalten.

Im Fall der Heißhomogenisationstechnik wird die Ciclosporinhaltige Lipidschmelze in einer heißen wäßrigen Emulgatorlösung durch Rühren dispergiert, z.B. durch den Gebrauch eines Rotor-Stator-Systems (Ultra Turrax® oder Silverson), oder durch die Anwendung eines Blattrührers, eines Propellerrührers, einer Zahnscheibe etc. Anstatt der Einarbeitung des Emulgators in die Wasserphase kann der Emulgator alternativ in die Lipidschmelze eingearbeitet werden. Dies ist besonders vorteilhaft im Fall eines lipophilen Emulgators oder Lecithins. Im Fall der Nutzung von zwei oder mehreren Emulgatoren können alle Emulgatoren in einer Phase (Wasser oder Lipid) oder in unterschiedlichen Phasen gelöst werden.

Die erhaltene O/W Voremulsion wird anschließend durch Hochdruckhomogenisation homogenisiert. Beispielsweise kann ein Kolben-Spalt-Homogenisator wie der Micron Lab 40, Lab 60 und/oder Gaulin 5,5 (APV-Homogenizer GmbH, Lübeck, Deutschland) bei Drücken typischerweise zwischen 100 bar und 1500 bar in einem oder mehreren Homogenisationszyklen eingesetzt werden. Die erhaltene Nanoemulsion wird abgekühlt, die flüssige Ölphase erhärtet und bildet Ciclosporin-beladene feste Lipidnanopartikel (SLN).

Im Fall des Einsatzes der Kalthomogenisationstechnik wird die arzneistoffenthaltende Schmelze abgekühlt. Die Emulgatoren werden genau so benutzt wie bereits bei der Heißhomogenisationstechnik beschrieben wurde. Das erhärtete Fett wird gemahlen, z.B. in einer Mörsermühle, um ein grobes Pulver zu erhalten. Falls erforderlich kann Trockeneis oder flüssiger Stickstoff zugesetzt werden, um die Sprödigkeit des Lipids während des Mahlvorgangs zu erhöhen. Das gemahlene Lipid wird in einer kalten Emulgatorlösung dispergiert und die erhaltene Lipid-Suspension im festen Zustand homogenisiert. Die Homogenisationstemperatur bleibt unterhalb der Schmelztemperatur des Lipids. Im Falle einer möglichen Hitzeentwicklung während des Herstellungsprozesses sollte eine Homogenisationstemperatur deutlich unterhalb des Schmelzpunktes des Lipids verwendet werden (z.B. durch Gegenkühlung), um ein Schmelzen des Lipids während des Homogenisationsvorganges zu verhindern. Bei der Hochdruckhomogenisation liegt die Temperatur in der Regel zumindest 5° unterhalb der Schmelztemperatur des Fettes. In den meisten Fällen wird die Kalthomogenisation bei Raumtemperatur durchgeführt, eine Kühlung unterhalb Raumtemperatur ist auch möglich.

Als dispergierte Phase können Lipide im weitesten Sinn als individuelle Komponente oder als Mischung angewendet werden.

Beispiele dafür sind: Natürliche oder synthetische Triglyceride bzw. Mischungen derselben, Monoglyceride und Diglyceride, alleine oder Mischungen derselben oder mit z.B. Triglyceriden, selbst-emulgierende modifizierte Lipide, natürliche und synthetische Wachse, Fettalkohole, einschließlich ihrer Ester und Ether sowie in Form von Lipidpeptiden, oder irgendwelche Mischungen derselben. Besonders geeignet sind synthetische Monoglyceride, Diglyceride und Triglyceride als individuelle Substanzen oder als Mischung (z.B. Hartfett), Imwitor 900, Triglyceride (z.B. Glyceroltrilaurat, Glycerolmyristat, Glycerolpalmitat, Glycerolstearat und Glyzerol-behenat) und Wachse wie z.B. Cetylpalmitat und weißes Wachs (DAB).

Der Anteil 1 der inneren oder Lipidphase bezogen auf die Gesamtformulierung ist 0,1% bis 40% (m/m) und liegt vorzugsweise im Bereich von 1% bis 20% (m/m). Sollte der Zusatz von dispersionsstabilisierenden Additiven notwendig oder gewünscht sein, z.B. Emulgatoren, um stabile Dispersion zu produzieren zu können, so können diese in Form von reinen Substanzen oder in Form von Mischungen eingearbeitet sein, um die Partikel zu stabilisieren. Die Menge an solchen Additiven, die im Verhältnis zu der gesamten Einwaage der wäßrigen Dispersion zugesetzt werden können, liegt im Bereich von 0,01% bis 20% und vorzugsweise im Bereich von 0,5% bis 10%.

Die folgenden Substanzen können als stabilisierende Zusätze gebraucht werden:
1. Tenside, im besonderen ethoxylierte Sorbitanfettsäure-Ester, Blockpolymere und Block-Copolymere (wie z.B. Poloxamere und Poloxamine), Polyglycerinether und Polyglycerinester, Lecithine unterschiedlicher Herkunft (z.B. Eilecithin oder Sojalecithin), chemisch modifizierte Lecithine (z.B. hydrierte Lecithine), genauso wie Phospholipide und Sphingolipide, Mischung von Lecithinen mit Phospholipiden, Sterolen (z.B. Cholesterol und Cholesterol-Derivate, genauso wie Stigmasterin), Ester und Ether von Zuckern oder von Zuckeralkoholen mit Fettsäuren oder Fettalkoholen (z.B. Saccharose-Monostearat);
2. sterisch stabilisierende Substanzen wie Poloxamere und Poloxamine (Polyoxyethylen-Polyoxypropylen-Block-Copolymere), ethoxylierte Sorbitanfettsäure-Ester, besonders Polysorbate (z.B. Polysorbat 80 bzw. Tween® 80), ethoxylierte Mono- und Diglyceride, ethoxylierte Lipide, ethoxylierte Fettalkohole oder Fettsäuren, und
3. geladene ionische Stabilisatoren und Peptisatoren so wie Diacetylphosphate, Phosphatidylglycerin, genauso wie gesättigte und ungesättigte Fettsäuren, Natriumcholat, Natriumglycocholat, Natriumtaurocholat oder ihrer Mischungen, Aminosäuren oder Peptisatoren, wie z.B. Natriumcitrat [J.S. Lucks, B. W. Müller, R. H. Müller, Int. J. Pharmaceutics 63, 183-188, 1990].
4. Viskos itätserhöhende Substanzen wie z. B. Cellulose-Ether und Cellulose-Ester (z.B. Methylcellulose, Hydroxyethylcellulose, Hydroxypropylcellulose, Natriumcarboxymethylcellulose), Polyvinylderivate sowie Polyvinylalkohol, Polyvinylpyrrolidon Polyvinylacetat, Alginate, Polyacrylate (z.B. Carbopol®), Xanthane und Pektine.

Die Ladungsstabilisatoren sind, wenn notwendig oder gewünscht, vorzugsweise in Bezug auf die Basisformulierung mit 0,01% bis 10% (m/m) inkorporiert und insbesondere in einer Menge von 0,05% bis zu 2%.

Viskositätserhöhende Substanzen sind, wenn notwendig oder erwünscht, im ähnlichen Verhältnis zur Basisformulierung eingearbeitet, vorzugsweise in einer Menge von 0,01-10% und insbesondere in einer Menge von 0,1% bis 10% (m/m) und vorzugsweise im Bereich zwischen 0,5% und 5%.

Als äußere Phase (= Dispersionsmedium, kontinuierliche Phase) können Wasser, wäßrige Lösungen oder Flüssigkeiten mischbar mit Wasser, sowie Glycerin oder Polyethylenglykol eingesetzt werden. Die wäßrigen Lösungen können für diesen Zweck nichtisotonisch oder isotonisch sein. Wäßrige Lösungen sind Mischungen von Wasser mit einer oder mehreren anderen Komponenten wie: Glycerin, Mannose, Glukose, Fruktose, Xylose, Trehalose, Mannitol, Sorbitol, Xylitol oder andere Polyole, sowie Polyethylenglykole, ebenso Elektrolyte wie beispielsweise Natriumchlorid. Diese Komponenten werden dann proportional zu der Basisformulierung in einer Menge von 0,1 Gew.% bis 50 Gew.% und vorzugsweise in einer Menge zwischen 1 Gew.% und 30 Gew.%, bezogen auf die Gesamtformulierung, zugesetzt.

Tensidfreie SLN werden hergestellt durch Dispergierung der Lipidphase in einer wässrigen Lösung, die eine oder mehrere viskositätserhöhende Substanzen enthält, entweder allein oder in Kombination mit anderen Substanzen, sowie Zucker, Zuckeralkohole, besonders Glukose, Mannose, Trehalose, Mannitol, Sorbitol sowie andere. Desweiteren ist es möglich, eine Kombination der viskositätserhöhenden Stoffe oder die Kombination dieser mit Zuckern oder Zuckeralkoholen, oder in einer weiteren Kombination mit Ladungsstabilisatoren oder Peptisatoren zu gebrauchen. Geeignete Peptisatoren sind beispielsweise Natriumcitrat, Natriumpyrophosphat, Natriumsorbat.

Die Einarbeitung von Ciclosporin in die Lipidmatrix kann nach unterschiedlichen Methoden erfolgen:
1. durch Lösen von Ciclosporin in der inneren Phase;
2. durch Lösen von Ciclosporin in einem Lösungsmittel, das mit der inneren Phase mischbar ist und der inneren Phase zugesetzt wird. Das Lösungsmittel wird anschließend, wenn erforderlich, teilweise oder komplett entfernt;
3. durch Dispergierung des Ciclosporins in der Lipidphase (z.B. durch die Dispergierung von feingemahlenem Ciclosporin (beispielsweise nano-große Cyclosporinkristalle) oder einer kontrollierten Präzipitation des Ciclosporins im Lipid oder Lipid-Lösungsmittel-Gemisch);
4. durch Lösen des Ciclosporins in der äußeren wäßrigen Phase und Einschluß der aktiven Substanz während des Produktionsprozesses in einem partikelstabilisierenden Tensidfilm;
5. durch Adsorption von Ciclosporin an die Oberfläche von Partikeln; (es sind Freisetzungsuntersuchungen durchgeführt worden, die zeigen, daß eine schnelle Freisezung von bis zu 20 % erfolgt ist, was anzeigt, daß zumindest ein Teil des Arzneistoffs auf der Oberfläche lokalisiert war).
6. durch Lösen des Ciclosporin in der Lipidphase mit Hilfe eines lösungsvermittelnden Zusatzes (z.B. einem BlockCopolymer oder einem Sorbitan-Fettsäureester) und anschließendem Dispergieren der Lipidphase um eine Prä-Emulsion oder eine Prä-Suspension herzustellen. Ciclosporin liegt dann in der Lipidmatrix als eine feste Lösung vor.

Sterilisation kann angewendet werden gemäß den Anweisungen des Arzneibuchs, z.B. durch Autoklavieren bei 110° oder γ-Bestrahlung oder anderen anerkannten Verfahren. Eine weitere mögliche Technik der Keimreduktion ist die Tyndalisierung.

Für die orale Applikation können die Ciclosporin SLN Dispersionen in ein Trockenprodukt überführt werden. Pulver können z.B. durch Sprühtrocknung oder Lyophilisation hergestellt werden. Die Endform ist ein mit Pulver gefülltes Sachet oder eine Weichgelatinekapsel, alternativ kann das Pulver auch zu Pellets oder Tabletten verarbeitet werden. Die Herstellung von Tabletten und Pellets kann auch ohne Zwischenschritt eines trockenen Pulvers erfolgen, indem die SLN Dispersion bei der Tablettierung als Granulierungsflüssigkeit oder beim Anteigen der Pelletmasse anstelle von Wasser eingesetzt wird.

Zur pulmonalen Applikation kann die SLN Dispersion in einem handelsüblichen Vernebler (z.B. Pariboy in Deutschland) vernebelt und direkt inhaliert werden. Alternativ kann das SLN Pulver in einen Pulverinhalator gefüllt werden oder in andere kommerziell verfügbare Inhalatoren.

Zur Herstellung von Salben oder Cremes mit Ciclosporin SLN kann eine vorhandene Salbe oder Creme mit einer konzentrierten SLN Dispersion (z.B. 20-30%) durch Rühren gemischt werden. Alternativ kann beim Herstellungsprozeß ein Teil des Wasser bei der Creme durch wäßrige SLN Dispersion ersetzt werden. Herstellung der Creme kann auch bei Temperaturen oberhalb des Schmelzpunktes der SLN erfolgen, da diese ausreichend physiklisch stabil sind. Eine Koaleszenz mit Öltropfen der Creme tritt bei ausreichend stabilisierten SLN nicht ein. Bei der Salbe ersetzt man analog einen Teil des Öls durch SLN Öldispersion.

Zur Herstellung von Gelen oder Lotionen wird die äußere Phase von SLN mit einem Gelbildner gebildet (z.B. Aerosil, Cellulosederivate wie Methyl- oder Hydroxyethylcellulose, z.B. Tylose H300 (Hydroxypropylcellulose mit einem Polymerisationsgrad von 400 und einem Molekulargewicht von 100 000)).

### Beispiel 1

Ciclosporin A beladene Lipidpartikel wurden hergestellt durch Lösen des Arzneistoffs und Tagat® S in der geschmolzenen Imwitor 900 Lipidmatrix bei 85 °C. Die heiße Schmelze wurde mit einem Rotor-Stator Rührer in einer wäßrigen Natriumcholat-Lösung bei 85 °C dispergiert und die erhaltene Voremulsion mit einem Micron LAB 40 (APV Homogenizer GmbH, Lübeck, Deutschland) homogenisiert. Die Hochdruckhomogenisation erfolgte in 3 Zyklen bei 500 bar und 85 °C. Die Formulierung enthielt 8% Imwitor 900, 2% Ciclosporin A (das entspricht 20% Cyclosporin bezogen auf die Lipidmatrix), 2,5 Tagat® S, 0,5% Natriumcholat und 87% destilliertes Wasser.

Diese Formulierung wurde in einer Dosierung von 16 mg/kg an drei Schweinen oral appliziert. Die Gabe der Zubereitung erfolgte nach Verdünnen der SLN Dispersion mit Wasser zu 40 ml über einem Magenkatheter, der Katheter wurde anschließend mit 200 ml Wasser gespült. Die Schweine wurden 4 h nach der oralen Gabe gefüttert. Die Blutspiegelkurven wurden abgebildet als Funktion der Zeit, die Gehaltsbestimmung von Ciclosporin erfolgte über einen validierten Immuno-Enzym-Assay (EMIT). Als Referenz wurde Sandimmun Neoral® (Novartis Pharma AG Basel, Schweiz) bei gleicher Dosierung in gleicher Art und Weise (Verdünnung zu 40 ml und orale Gabe über den Magenkatheter, spülen mit 200 ml Wasser) angewendet. Der mittlere Blutspiegel der 3 Versuchstiere für jede der beiden Zubereitungen wird in Abb. 2 gezeigt (oben: Sandimmun Neoral® als Referenz, unten: Ciclosporin A-beladene feste Lipidnanopartikel).

### Beispiel 2

Feste Lipidnanopartikel (SLN) Dispersionen mit ansteigender Ciclosporin-Beladung (5%, 10%, 15%, 20%) wurden mit Hilfe der Heißhomogenisationstechnik hergestellt. Die Produktion erfolgte durch Schmelzen der Lipidphase bei 85 °C, lösen des Arzneistoffs und Tagat® S in der Schmelze durch Rühren, Zusatz der arzneistoffbeladenen Schmelze zu einer wäßrigen Natriumcholat-Lösung und Herstellung einer Voremulsion durch Rühren mit einem Rotor-Stator (Ultra Turrax® T 25 einschließlich der Dispergiereinheit N 18 G, Jahnke & Kunkel, Stauffen, Deutschland). Die Voremulsion wurde bei 85 °C mit einem temperaturkontrollierten Micron LAB 40 Kolben-Spalt Homogenisator (APV Homogenizer GmbH, Lübeck, Deutschland) bei 500 bar in 3 Homogenisationszyklen homogenisiert. Die Zusammensetzung der SLN Formulierungen sind in Tab. 2 aufgeführt.

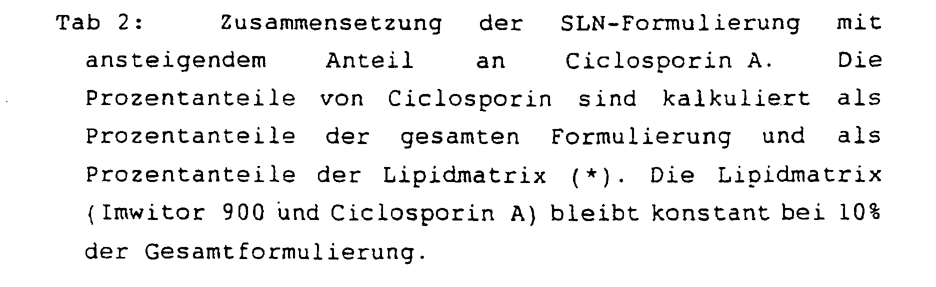

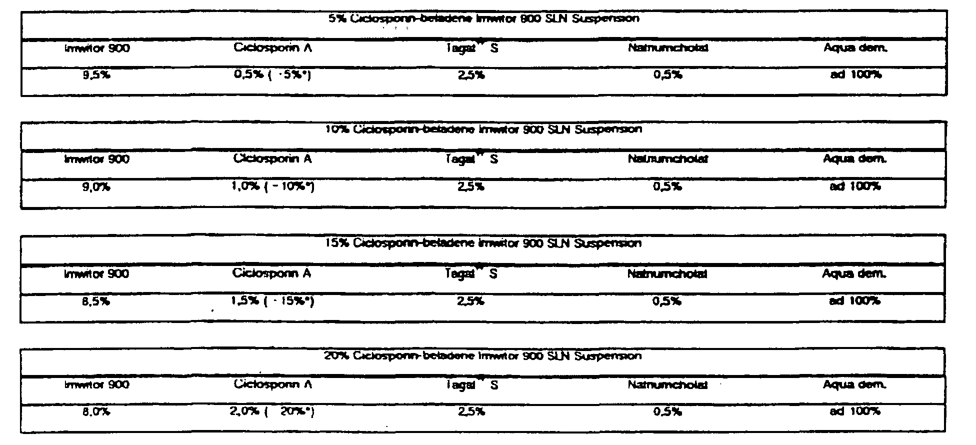

Die Partikelgrößen wurden mit Hilfe der Laserdiffraktometrie (LD) mit einem Mastersizer E (Malvern Instruments, England) bestimmt. Die LD Durchmesser d50%, d95% und d99% wurden ausgewählt, um die Feinheit der Partikeldispersionen zu bestimmen und zu charakterisieren. Die Partikelgrößenwerte sind in Tab. 3 aufgelistet, die Größenverteilungskurven in Abb. 4a dargestellt.

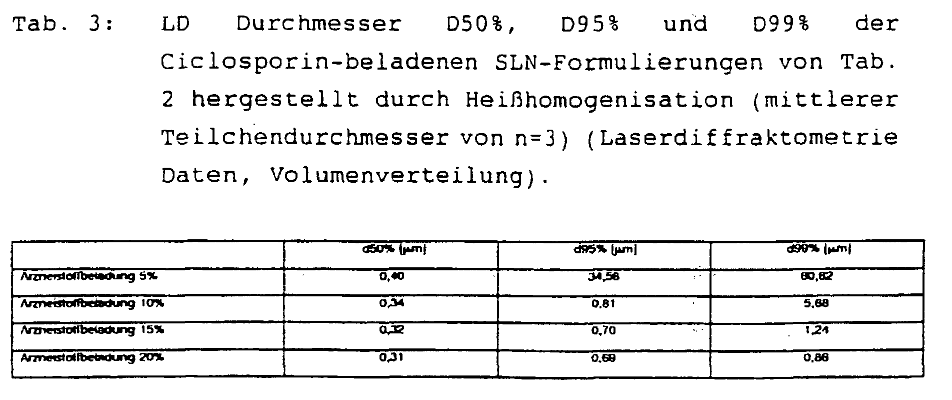

### Beispiel 3

Feste Lipidnanopartikel (SLN) Dispersionen mit ansteigender Ciclosporin A-Beladung (5%, 10%, 15%, 20%) wurden identisch in der Zusammensetzung zu Beispiel 2 (Tab. 2) produziert, jedoch durch Anwendung der Kalthomogenisationstechnik. Nach dem Lösen des Arzneistoffs und des Tagat S® in der Lipidschmelze wurde die Schmelze abgekühlt und in einer Mörsermühle für 10 min gemahlen (Mörsermühle, Fa. Retsch, Hahn, Deutschland). Die erhaltenen groben Partikel wurden in einer wäßrigen Natriumcholat-Lösung mit Hilfe eines Ultra Turrax® dispergiert. Die erhaltene Suspension wurde bei 55° homogenisiert, d.h. etwa 5 °C unterhalb des Schmelzbereichs von Imwitor 900 (Schmelzbereich 59 °C bis 61 °C). Ein temperaturkontrollierter Micron Lab 40 (APV Homogenizer GmbH, Lübeck, Deutschland) wurde verwendet und die Homogenisation fand in 3 Homogenisationszyklen bei Raumtemperatur statt. Die Partikelgrößen wurden mit Hilfe der Laserdiffraktometrie (LD) ermittelt (Mastersizer E, Malvern Instruments, England). Die LD Durchmesser d50%, d95% und d99% wurden zur Charakterisierung der Feinheit der Partikel der Dispersion herangezogen und sind in Tab. 4 gezeigt, die korrespondierenden Größenverteilungskurven in Abb. 4b.

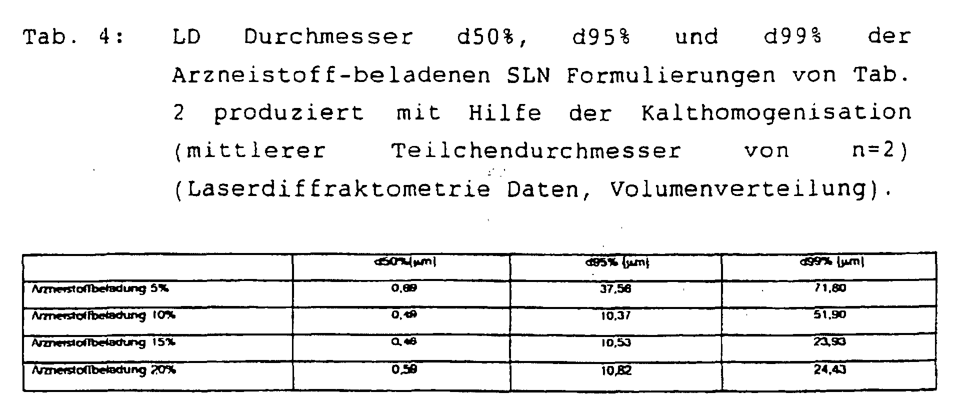

### Beispiel 4

Physikalische Lipid-Arzneistoff-Mischungen von Imwitor 900 und Ciclosporin A wurden mit steigendem Anteil an Ciclosporin A von 0%-30% hergestellt. Ciclosporin A wurde in Imwitor 900 durch Erwärmen für 15 min auf 85 °C gelöst, anschließend die Lipidmatrix mit inkorporiertem gelöstem Arzneistoff wieder abgekühlt. Die DSC Aufheizkurven dieser so erhaltenen Mischungen zeigen ein Anwachsen der Fraktion der weniger stabilen α-Modifikation (Abb. 6).

### Beispiel 5

Die Einkapselungsrate wurde mit Hilfe der HPLC-Analytik bestimmt, die SLN-Partikel waren hierbei mit ansteigendem Ciclosporin A Gehalt beladen. Die Partikel wurden mit Hilfe der Heißhomogenisationstechnik in 3 Homogenisationszyklen bei 500 bar und 85 °C hergestellt (Zusammensetzung: siehe Tab. 2). Die Einkapselungsrate ist definiert als der Prozentsatz der totalen Arzneistoffkonzentration in der Formulierung, der innerhalb der Partikel eingeschlossen ist (100% Arzneistoff in der Formulierung sind gleich X Prozent im Lipidpartikel plus Y Prozent freier Arzneistoffanteil in der wäßrigen Phase). Die Daten sind in Tab. 5 aufgelistet.

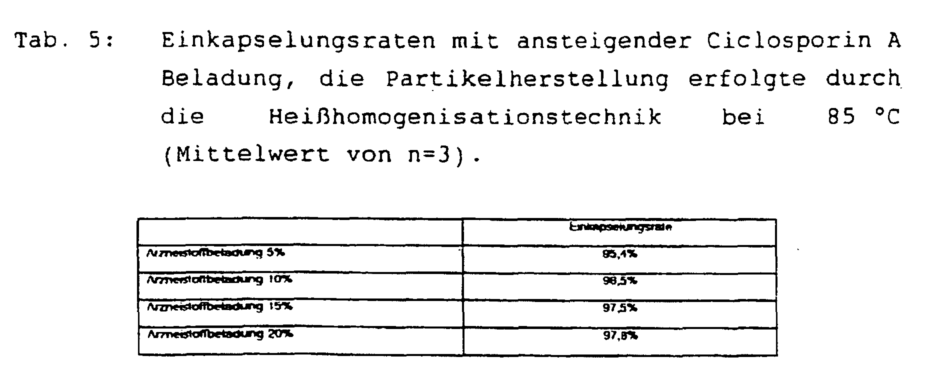

### Beispiel 6

Die Einkapselungsrate wurde bestimmt mit Hilfe der HPLC-Analyse von SLN-Partikeln mit ansteigendem Arzneistoffanteil in der Lipidmatrix, die Partikel wurden hergestellt durch die Kalthomogenisationstechnik bei 3 Zyklen und 55 °C (Zusammensetzung: siehe Tab. 2). Die Daten sind in Tab. 6 aufgelistet.

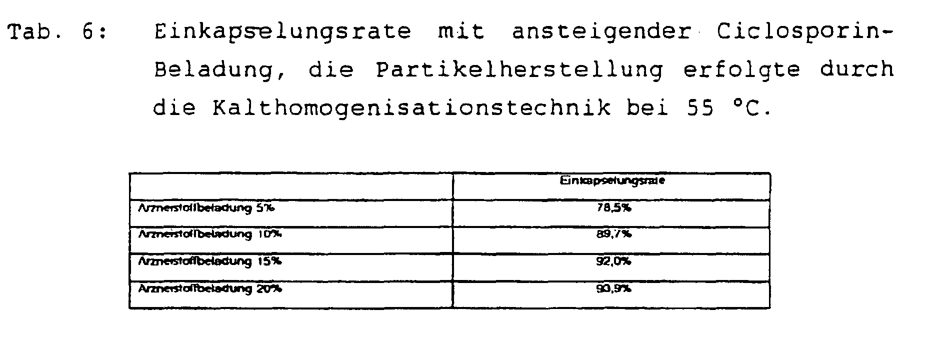

### Beispiel 7

Eine wäßriger Lösung von 10% Trehalose (m/m) wurde im Verhältnis 1:1 mit einer wäßrigen festen Lipidpartikel Dispersion (8% Imwitor 900, 2% Ciclosporin A, 2,5% Tagat S®, 0,5% Natriumcholat, 87% destilliertes Wasser) gemischt und gefriergetrocknet. Der Einfrierprozeß wurde bei -20 °C in Injektionsflaschen mit 2 ml der Trehalose-Lipidpartikel-Mischung durchgeführt, die Gefriertrocknung erfolgte in einer Gamma-2-20 Anlage der Fa. Christ, Osterode i.H., Deutschland für 24 h bei -10 °C und einem Vakuum von 0,370 bar. Die Nachtrocknung der Suspension fand bei 0 °C, 370 mbar in einem Zeitraum von 12 h statt. Das Produkt war ein trockenes und flockiges Pulver, Ciclosporin war in der Lipidmatrix in einem amorphen Zustand eingekapselt, wie mit Hilfe der Weitwinkelröntgendiffraktometrie nachgewiesen werden konnte. Die Röntgenstrukturanalyse des Produktes erfolgte am Tag 1 nach Gefriertrocknung und am Tag 180 nach Lagerung bei 25 °C (Abb. 8). Nach 180-tägiger Lagerung kristallisierte lediglich Trehalose aus, wie deutlich an den Streupeaks in Winkelbereichen von 2 Theta = 13° und höher erkennbar ist. Ciclosporin verblieb über den gesamten Lagerungszeitraum in einem amorphen Zustand, was deutlich an der Abwesenheit von typischen Ciclosporin Kristallpeaks im Streumuster in Abb. 8 erkennbar wird (2 Theta = 7° bis 13°).

### Beispiel 8

Ein Betrag von 10% Trehalose (m/m) in einer 10%-igen ethanolischen wässrigen Lösung wurde im Verhältnis 1:1 mit einer wäßrigen festen Lipidpartikel Dispersion (8% Imwitor 900, 2% Ciclosporin A, 2,5% Tagat S®. 0,5% Natriumcholat, 87% destilliertes Wasser) vermischt. Die erhaltene Mischung wurde an einem Minibüchi (Büchi, Schweiz) sprühgetrocknet. Die Sprühtrocknungsparameter waren: 95 °C Inlet Temperatur, 45 °C Outlet Temperatur, 1 ml/min Flußrate. Das Produkt war ein trocknes und flockiges Pulver. Ciclosporin war in die Lipidmatrix in einer amorphen Form eingekapselt.

### Beispiel 9

Zubereitungen von 2 kg SLN Dispersionen wurden an einem modifizierten Lab 60 Kolben-Spalt Homogenisator (APV Homogenizer GmbH, Lübeck, Germany) produziert. Der Homogenisator verfügte über temperierbare Gefäße, Leitungen und Homogenisationsventile wie beschreiben in [R.H. Müller, S. Gohla, G.E. Hildebrand, S.A. Runge, A. Dingler, Dispersion of solid lipids - solid lipid nanoparticles (SLN): Production and possible applications in food, cosmetic and pharmaceutical products, World Congress on Emulsion, Bordeaux, 1-2-195, 1997]. Der 10 kg Zulaufbehälter war mit einer Zahnscheibe ausgestattet, der Produktauffangbehälter mit einem 4-blättrigen Edelstahl-Propellerrührer. Die Produktion erfolgte im kontinuierlichen Umlaufbetrieb, d.h. nach dem Passieren des Homogenisationsspaltes und erfolgter Homogenisation wurde das fein dispergierte Produkt in den Zulaufbehälter direkt zurückgeführt, um eine erneute Homogenisation zu ermöglichen.

40,0 g Ciclosporin A (2.0%) wurden bei 85 °C in 160,0 g geschmolzenem Imwitor 900 (8,0%) mit 50,0 g Tagat S® (2,5%) unter Rühren gelöst. Die Schmelze von 250,0 g wurde in 1750 g Wasser mit 10,0 g Natriumcholat (0,5%) dispergiert. Bei 85 °C erfolgte die Homogenisation mit 500 bar im kontinuierlichen Umlaufbetrieb über 20 min. Die Formulierungen wurden mit ansteigenden Homogenisationszeiten in 5 min.-Stufen zubereitet (Tab. 7). Die Partikelgrößenanalyse erfolgte mittels der Laserdiffraktometrie (Mastersizer E, Malvern Instr., UK) und Photonenkorrelationsspektroskopie (Coulter N4Plus, Coulter Electr., USA).

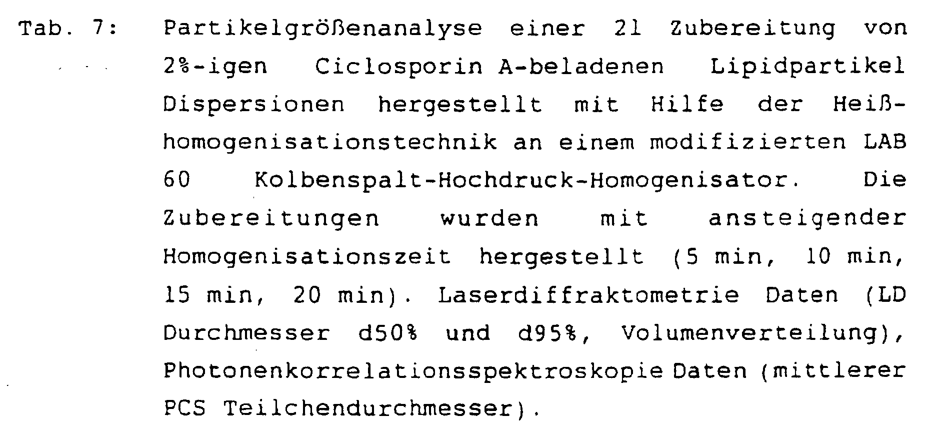

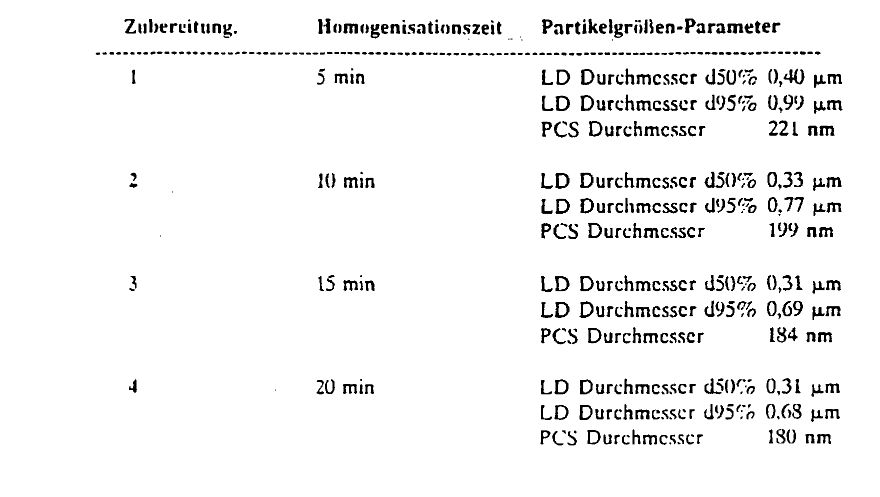

### Beispiel 10

40,0 g Ciclosporin (=2%) wurden bei 80 °C in 160,0 g geschmolzenen Imwitor 900 (8,0%) inklusive 50,0 g Tagat S® (2,5%) durch Rühren gelöst. Die Schmelze von 250,0 g wurde in 1750 g Wasser inklusive 10,0 g Natriumcholat (0,5%) dispergiert. Die Emulsion wurde mit einer Zahnscheibe für 1 min bei 150 U/min bei 80 °C dispergiert (Tab. 8). Die Partikelgrößenanalyse wurde mit Hilfe der Laserdiffraktometrie und Photonenkorrelationsspektroskopie durchgeführt (Mastersizer E für die Laserdiffraktometrie, Malvern Instruments, England und Coulter N4 Plus für die Photonenkorrelationsspektroskopie, Coulter Electronics, USA).

**Tab. 8:**

| Die Partikelgrößenanalyse einer 2 1 Zubereitung einer 2% Ciclosporin A-beladenen Lipidpartikel Dispersion nach Rühren mit einer Zahnscheibe (ø = 15 cm) bei 85 °C mit 150 U/min. Laserdiffraktometrie Daten (LD Durchmesser d50% und d95%, Volumenverteilung), Photonenkorrelationsspektroskopie Daten (mittlerer PCS Teilchendurchmesser). | |
|---|---|
| LD Durchmesser d50% | 0,83 µm |
| LD Durchmesser d95% | 19,33 µm |
| PCS Durchmesser | 322 nm |

### Beispiel 11

800 mg Ciclosporin wurden bei einer Temperatur von 85 °C in 3200 mg geschmolzenem Compritol 888 ATO durch Rühren gelöst. Die Schmelze (4,0 g) wurde in 36,0 g Wasser dispergiert. Die wäßrige Lösung beinhaltete Polysorbat 80 und Sojalecithin (Lipoid S75). Die Emulgator-Konzentration war 1360 mg bzw. 120 mg (kalkuliert als Anteil der Gesamtformulierung von 40,0 g, entsprechend 3,4% für Polysorbat 80 und 0,3% für Lipoid S 75). Bei 85 °C wurde mit Hilfe der Heißhomogenisation bei einem Druck von 500 bar und 3 Homogenisationszyklen die Lipiddispersion homogenisiert (Tab. 9). Die Partikelgrößenanalyse wurde mit Hilfe der Laserdiffraktometrie und Photonenkorrelationsspektroskopie durchgeführt (Mastersizer E für die Laserdiffraktometrie, Malvern Instruments, England und Coulter N4 Plus für die Photonenkorrelationsspektroskopie, Coulter Electronics, USA).

**Tab. 9:**

| Partikelgrößenanalyse einer 2% Ciclosporinbeladenen Lipidpartikel Dispersion mit Compritol 888 ATO als Lipidmatrix (8%), Ciclosporin (2%), Polysorbat 80 (3,4%) und Sojalecithin (Lipoid S 75, 0,3%) als Emulgatoren in destilliertem Wasser (86,3%). Laserdiffraktometrie Daten (d50%, d95% und d99%, Volumenverteilung), Photonenkorrelationsspektroskopie Daten (PCS mittlerer Teilchendurchmesser, Polydispersitätsindex (PI). | |
|---|---|
| LD Durchmesser d50% | 0,33 µm |
| LD Durchmesser d95% | 0,91 µm |
| LD Durchmesser d99% | 3,00 µm |
| PCS Durchmesser | 163 µm |
| PCS Polydispersitätsindex | 0,339 |

### Beispiel 12

800 mg Ciclosporin wurden bei 85 °C in 3200 mg geschmolzenem Precirol ATO 5 als Lipidmatrix durch Rühren gelöst. Die Schmelze von 4,0 g wurde dispergiert in 36,0 g Wasser mit Poloxamer 188 und Natriumcholat (kalkuliert auf die Gesamtformulierung von 40,0 g, entsprechend 2,5% Poloxamer 188 und 0,5% Natriumcholat) als Emulgatoren. Die Heißhomogenisation und Partikelgrößenanalyse (Tab. 10) wurde entsprechend dem Beispiel 11 durchgeführt.

**Tab. 10:**

| Partikelgrößenanalyse einer 2% Ciclosporinbeladenen Lipidpartikel Dispersion mit Precirol ATO 5 als Lipidmatrix (8%), Ciclosporin (2%), Poloxamer 188 (2,5%) und Natriumcholat (0,5%) als Emulgatoren in destilliertem Wasser (87%). Laserdiffraktometrie Werte (d50%, d95% und d99%, Volumenverteilung), Photonenkorrelationsspektroskopie Daten (mittlerer PCS Teilchendurchmesser, Polydispersitätsindex (PI)). | |
|---|---|
| LD Durchmesser d50% | 0,30 µm |
| LD Durchmesser d95% | 0,74 µm |
| LD Durchmesser d99% | 5,73 µm |
| PCS Durchmesser | 193 nm |
| PCS Polydispersitätsindex | 0,312 |

### Beispiel 13

800 mg Ciclosporin wurden bei 85 °C in 3200 mg geschmolzenem Bienenwachs durch Rühren gelöst, die Schmelze von 4,0 g wurde in 36,0 g Wasser mit Polysorbat 80 als Emulgator dispergiert. Die Emulgatorkonzentration betrug 480 mg (kalkuliert auf die Gesamtformulierung von 40,0 g entspricht das 1,2%). Die Heißhomogenisation und Partikelgrößenanalyse (Tab. 11) wurde entsprechend den Ausführungen in Beispiel 11 durchgeführt.

**Tab. 11:**

| Partikelgrößenanalyse einer 2% Ciclosporinbeladenen Lipidpartikel Dispersion mit Bienenwachs als Lipidmatrix (8%), Ciclosporin (2%) und Polysorbat 80 (1,2%) als Emulgator in destilliertem Wasser (88,8%). Laserdiffraktometrie Daten (d50%, d95% und d99%, Volumenverteilung) Photonenkorrelationsspektroskopie Daten (mittlerer PCS Teilchendurchmesser, Polydispersitätsindex (PI) ). | |
|---|---|
| LD Durchmesser d50% | 0,36 µm |
| LD Durchmesser d95% | 1,20 µm |
| LD Durchmesser d99% | 3,51 µm |
| PCS Durchmesser | 279 nm |
| PCS Polydispersitätsindex | 0,148 |

### Beispiel 14

### Herstellung von SLN mit Tetracain

Zur Herstellung von mit Tetracain beladenen Lipidpartikeln wurden 0,40 g Tetracain in 3,60 g geschmolzenem Imwitor 900 gelöst (= 10 % Arzneistoff in der Lipidphase) , 1,00 g Tagat S der Schmelze zugesetzt und diese dann in 35,0 g heißem Wasser, dem 0,20 g Natriumcholat zugesetzt war, mit einem Rotor-Stator dispergiert. Die Gesamtrezeptur enthielt somit 10 % Lipidphase (9 % Lipid und 1 % Tetracain). 2,5 % Tagat S, 0,5 % Natriumcholat, und 87 % Wasser. Die erhaltene Rohemulsion wurde mit einem LAB 40 bei 85 °C und einem Druck von 500 bar mit 3 Zyklen homogenisiert. Nach Abkühlen bildeten sich feste Lipidpartikel. Partikelgrößenanalytik mit einem Laserdiffraktometer (MasterSizer E, Malvern Instruments) ergab einen Durchmesser 50 % von 0,39 µm, Durchmesser 95 % von 65 µm und Durchmesser 99 % von 77 µm.

Zur Herstellung von mit Tetracain beladenen Lipidpartikeln mit 20 % Arzneistoff in der Lipidphase wurde 0,80 g Tetracain in 3,20 g geschmolzenem Imwitor 900 gelöst und dann wie oben verfahren. Die Partikelgrößenanalytik ergab einen Durchmesser 50 % von 0,44 µm, Durchmesser 95 % von 71 µm und Durchmesser 99 % von 78 µm. Erhöhung des Arzneistoffgehalts erhöht die Polydispersität.

Zur Herstellung von mit Tetracain beladenen Lipidpartikeln unter Verwendung von Compritol als Lipid wurde identisch zur Herstellung von mit Tetracain beladenen Imwitor 900 Partikeln verfahren. Bei 10 % Arzneistoffgehalt in der Lipidphase ergaben sich ein Durchmesser 50 % von 0,45 µm, Durchmesser 95 % von 75 µm und Durchmesser 99 % von 79 µm.

Bei 20 % Arzneistoffgehalt in der Lipidphase ergaben sich ein Durchmesser 50 % von 0,39 µm, Durchmesser 95 % von 73 µm und Durchmesser 99 % von 79 µm.

Ersatz von Cyclosporin durch Tetracain führte zu extrem polydispersen Partikelpopulationen, bei 10 % Arzneistoff in der Lipidphase liegen 95 % der Partikel in einem breiten Bereich von 0,1 - 65 bzw. 75 µm bei Imwitor 900 bzw. Compritol als Lipid (Vergleich Cyclosporin aus Beispiel 2: 0,1 - 0,8 µm).

Die Erhöhung des Arzneistoffanteils führte zu keiner Verbesserung der Homogenität wie sie z.B. bei Cyclosporin-Partikeln beobachtet wurde, der Durchmesser 99 % bleibt unverändert bei ca. 78 µm (Vergleich Cyclosporin aus Beispiel 2, bei Erhöhung von 10 % auf 20 % Cyclosporin Erniedrigung des Durchmessers 99 % von 5,68 Mm auf 0,86 µm).

### Problematik der Ciclosporin-Therapie und des notwendigen Drug Monitoring:

Die Bestimmung der Pharmakokinetik von Ciclosporin ist sowohl abhängig von der Art des biologischen Mediums (Blut vs. Plasma oder Serum) als auch von der Assay-Methode (radioimmunoassay (RIA) vs. Hochdruckflüssigkeitschromatographie (HPLC)). Aufgrund dieser Abhängigkeiten sind die Interpretation von pharmakokinetischen Daten und die Bestimmung einer Korrelation zwischen Konzentration in biologischen Flüssigkeiten und therapeutischen und/oder toxischen Effekten dieses Arzneistoffes sehr schwierig. Die am häufigsten auftretende und klinisch wichtigste Nebenwirkung von Ciclosporin ist die Dosis-abhängige Nephrotoxizität. Höhere Blutspiegel von Ciclosporin führen zu einem Anfluten höherer Konzentrationen des Arzneistoffs in den Nieren, was zu einer Vielzahl unterschiedlicher histologischer Veränderungen führt. Aufgrund dieser häufigen Komplikation erfordert die Ciclosporin-Therapie zwingend eine Individualisierung der Dosierung über intensives teures und zeitaufwendiges Monitoring der Blutspiegel und renalen Funktion. Mediziner in der Klinik führen ein Monitoring regelmäßig durch, das heißt während der frühen Posttransplantations-Periode täglich oder 3-4 mal pro Woche, nach 6 Monaten bis 1 Jahr nach Transplantation Reduzierung auf 1 mal monatlich. Das Monitoring erfolgt dabei im allgemeinen anhand der sogenannten Trough Blutspiegel, das heißt dem auslaufenden Blutspiegel nach dem ersten Plasmapeak (z.B. 24 Stundenwert).

### Dosierung, Blutspiegel, therapeutische Effizienz und Toxizität von Ciclosporin:

Maximale Blutspiegelkonzentrationen (Peaks) von Ciclosporin (bestimmt mit HPLC) sind ungefähr 1,4-2,7 ng/ml pro 1 mg von einer peroral applizierten Dosis von einer konventionellen Formulierung (z.B. Sandimmun) in gesunden Erwachsenen. Die Formulierung von Ciclosporin als eine Emulsion (Sandimmun Neoral/ Optoral) hat eine höhere Bioverfügbarkeit, was zu höheren Blutspiegelpeaks und zu einer größeren Fläche unter der Arzneistoffko-nzentration/Zeit-Kurve führt (AUC).

Mit RIA gemessene Trough Blutspiegelkonzentrationen (nach 24 Stunden) von 250-800 ng/ml scheinen sowohl die Häufigkeit der Organabstoßung als auch Ciclosporin induzierte Nebenwirkungen zu minimieren. Eine Verbindung zwischen Trough Serumkonzentrationen (gemessen mit RIA) oberhalb von 500 ng/ml (korrespondierende Blutkonzentrationsbereich 700-1350 ng/ml) und Ciclosporin-indizierter Nierentoxizität wurde berichtet.

Tabelle 12 gibt eine Zusammenfassung von klinischen Human-Daten, die den Effekt der Dosis auf die (mit RIA gemessene) maximale Blutspiegelkonzentration zeigt (nach Daten der AHFS, American Hospital Formulary Service, 1997, pp. 2862-2873). Zu Vergleichszwecken sind die Daten der an Schweinen durchgeführten Studie eingeschlossen um den Effekt der Erfindung zu demonstrieren.

### Beispiel 15

Ciclosporin A beladene Lipidpartikel wurden hergestellt wie in Beispiel 1. Die Formulierung enthielt 16% Imwitor 900, 4% Ciclosporin A, 2,5% Tagat S, 0,5% Natriumcholat und 77% Wasser (d.h. 20% Lipidpartikel in der Dispersion). 20 Teile der Lipidpartikel-Dispersion wurden unter Rühren schrittweise eingearbeitet in 80 Teile Basiscreme (Deutscher Arzneimittel-Codex (DAC) 1979, Govi-Verlag GmbH, Frankfurt/Main, Deutschland). Die Einarbeitung erfolgte mit Rührschale und Pistill bei Raumtemperatur. Die Creme enthielt einen Anteil von 4% Ciclosporin-Lipidpartikel.

### Beispiel 16

Ciclosporin A beladene Lipidpartikel wurden hergestellt wie in Beispiel 12. 100 g der wäßrigen Lipidpartikel-Dispersion wurden 1,0 g Tylose H300 (Hydroxethylcellulose, Polymerisationsgrad 400, Molekulargewicht 100 000) und 10,0 g Glycerol (87% in Wasser) zugesetzt. Die Tylose H300 wurde mit Rührschale und Pistill mit Glycerol angerieben. Nach feiner Verteilung von Tylose H300 in Glycerol wurde schrittweise die Lipidpartikel-Dispersion zugegeben. Nach einer Quellzeit entstand ein Gel.

### Beispiel 17

Ciclosporin A beladene Lipidpartikel wurden hergestellt wie in Beispiel 12. 100 g Basiscreme wurden hergestellt wie im DAC beschrieben, wobei 10 g Wasser durch wäßrige Lipidpartikel-Dispersion ersetzt wurde. Die bei erhöhter Temperatur oberhalb des Schmelzpunktes der Lipidpartikel hergestellte Creme enthält feste Lipidpartikel (Schmelzpeak in der Analyse und Differential Scanning Calorimetry).

### Beispiel 18

Ciclosporin (0,5%) wurde in geschmolzenem Compritol (4%) gelöst, in 80°C heißem Miglyol 812 unter Zusatz von Span 80 (1,2%) mit einem Ultra-Turrax dipergiert und hochdruckhomogenisiert (500 bar, 3 Zyklen bei 80°C). Zur Herstellung einer Salbe wurden nach Abkühlen 0,3 g Aerosil 200 mit 30 g der Lipidpartikel-Dispersion angerieben.

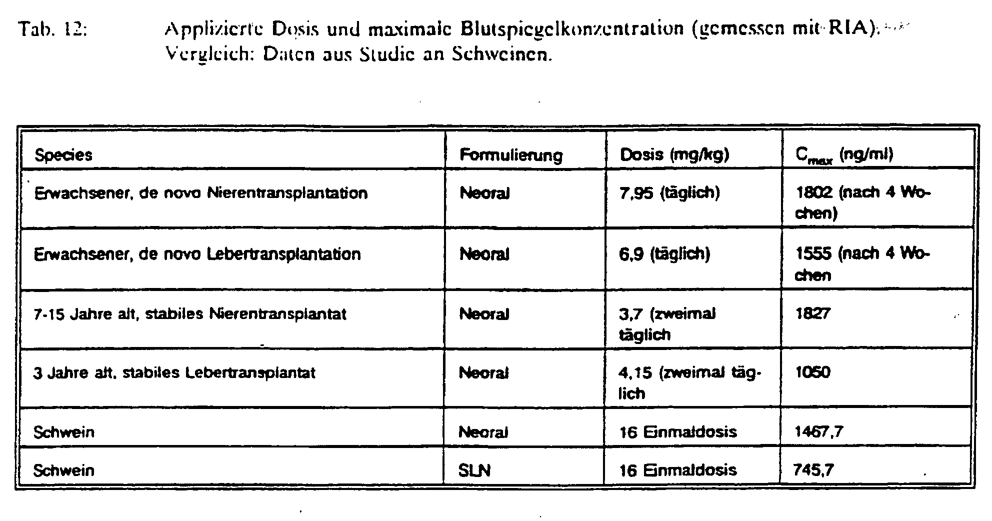

Die Applikation einer durchschnittlichen Erhaltungsdosis von Ciclosporin (z.B. 8 mg/kg) für einen 70 kg schweren erwachsenen Patienten mit existierenden Trough Blutspiegelwerten von 700 - 1350 ng/ml würde in einer maximalen Blutspiegelkonzentration (Peak) zwischen 1484 ng/ml und 2863 ng/ml resultieren (berechnet auf der Basis von HPLC-Daten für Sandimmun, Daten laut Hersteller). Berücksichtigt man die niedrigere Sensitivität von HPLC im Vergleich zu RIA und die gleichzeitig höhere Bioverfügbarkeit von Neoral, so können noch höhere Blutspiegelmaxima für Neoral angenommen werden. Basierend auf diesen Daten können toxische Blutspiegelwerte für Ciclosporin oberhalb von 1500 ng/ml angenommen werden. Die Applikation höherer Erhaltungsdosen zur Erzielung eines therapeutisch vorteilhaften höheren Trough Blutspiegels würde bei den bisherigen Formulierungen Peaks bis weit in den toxischen Blutspiegelbereich ergeben.

Klinische Vorteile der Erfindung: Die Abb. 2 und die von der Schweinestudie erhaltenen pharmakokinetischen Daten zeigen, daß man als eine überraschende und einmalige Eigenschaft der Erfindung eine dramatische Reduktion (50 %) und eine Verzögerung der maximalen Blutkonzentrationen im Vergleich zu Neoral bei identischer intra-gastrinaler Dosierung erhält. Die Trough Blutspiegel für beide Präparate sind ähnlich und die AUC Werte sind vergleichbar innerhalb der pharmazeutischen Bioäquivalenz-Grenzwerte. Es ist allgemein anerkannt, daß die gastrointestinale Anatomie des Schweins der des Menschen vergleichbar ist und die Daten von Studien in Schweinen in adäquaterweise den grundsätzlichen Verlauf im Menschen widerspiegeln. Die mit der erfindungsgemäßen Formulierung erzielte stark erniedrigte maximale Blutspiegelkonzentration und die Verzögerung ihres Entritts ist aus den folgenden Gründen überaus vorteilhaft für die Ciclosporin-Therapie:
1. Signifikant reduziertes Risiko für Nierentoxizität
2. Höhere Flexibilität in der Erhöhung der Dosierung aufgrund erniedrigter bzw. ausbleibender Plasmapeaks.
3. Die Dosiserhöhung ermöglicht erstmalig die Einstellung höherer Trough Blutspiegel, was zur Verminderung von Abstoßungsreaktionen führt (höhere therapeutische Effizienz).
4. Reduzierung der Häufigkeit von bisher notwendigem
   - teurem und zeitintensivem - regelmäßigem Monitoring von Ciclosporinblutspiegelkonzentrationen und regelmäßiger Überprüfung der renalen Funktion.

Die oben genannten Vorteile der erfindungsgemäßen Formulierung sind von speziellem Vorteil in der pädiatrischen Therapie mit Ciclosporin, und zwar nicht nur zur Verhinderung der Organabstoßung sondern auch für eine Reihe von entzündlichen schwer therapierbaren Erkrankungen (z.B. Morbus Crohn, ulzerierende Colitis, Psoriasis und juvenile Arthritis) mit einer signifikanten Häufigkeit (z.B. über 1 Million) in den USA.

## Patentansprüche

1. Arzneistoffträger, der feste Lipidpartikel beleden mit Ciclosporin oder Ciclosporinderivaten natürlichen und/oder synthetischen Ursprungs umfaßt, wobei die Partikel einen Teilchendurchmesser von 10 nm bis 10 µm aufweisen, durch Hochdruckhomogenisation hergestellt worden sind und in einem bei Raumtemperatur festem Zustand vorliegen, wobei die Partikel der Hauptpopulation einen mitleren PCS (miltels Photonencorrelations spektroskopie bestimmten) Teilchendurchmesser im Bereich von 40 nm bis 1000 nm besitzen.

2. Arzneistoffträger nach Anspruch 1, der tensidhaltige oder tensidfreie Partikel eines Lipids oder der Mischung von Lipiden umfaßt, die herstellbar sind, indem entweder eine innere Phase (Lipidphase) in einem Dispersionsmedium (Wasser, wäßrige Lösung oder eine mit Wasser mischbare Flüssigkeit) in geschmolzener oder erweichter Form dispergiert wird, oder eine innere Phase (Lipidphase) in einem Dispersionsmedium in einer festen Form dispergiert wird, wobei die feste Phase hierbei vor dem Dispergierprozeß fein zerkleinert wird.

3. Arzneistoffträger nach Anspruch 2, **dadurch gekennzeichnet, daß** die Partikel der Hauptpopulation einen mittleren Teilchendurchmesser im Bereich zwischen 100 nm und 500 nm besitzen und daß mit geeigneten ausgewählten Prozeßparametern und Zusätzen die PCS Teilchendurchmesser im Bereich zwischen 40 nm und 100 nm liegen.

4. Arzneistoffträger nach einem der Ansprüche 1 bis 3, **dadurch gekennzeichnet, daß** der Gehalt der inneren Phase (Lipidphase) bezogen auf die Gesamtformulierung im Bereich von 0,1% bis 40% (m/m) und insbesondere im Bereich zwischen 1% bis 20% (m/m) liegt.

5. Arzneistoffträger nach einem der Ansprüche 1 bis 4, **dadurch gekennzeichnet, daß** er als Material der Partikelmatrix Monoglyceride, Diglyceride und/oder Triglyceride, Fettalkohole, deren Ester oder Ether, Wachse oder Lipidpeptide oder eine Mischung derselben aufweist.

6. Arzneistoffträger nach einem der Ansprüche 1 bis 5, **dadurch gekennzeichnet, daß** er Glyceroltrilaurat, Glycerolmyristat, Glycerolpalmitat, Glycerolstearat und/oder Glycerolbehenat, oder eine Mischung derselben mit Mono-, Di- und Triglyceriden, als Glycerid-Mischung, insbesondere Imwitor 900, Fettalkohole, insbesondere Cetylalkohol und Stearylalkohol, und/oder Wachse, insbesondere Cetylpalmitat und gebleichtes Bienenwachs, beinhaltet.

7. Arzneistoffträger nach einem der Ansprüche 1 bis 6, **dadurch gekennzeichnet, daß** er einen Zusatz von einem oder mehreren dispersionsstabilisierenden Zusätzen in einer Menge bezogen auf die Gesamtformulierung von 0,01% bis 30% (m/m) und insbesondere im Bereich von 0,5 bis 10% (m/m) aufweist.

8. Arzneistoffträger nach Anspruch 7, **dadurch gekennzeichnet, daß** die dispersionsstabilisierenden Zusätze Substanzen aus der Reihe der Poloxamere, Poloxamine, ethoxylierten Monoglyceride und Diglyceride, ethoxylierten Lipide, ethoxylierten Fettalkohole und Alkylphenole, ethoxylierten Fettsäureester, Polyglycerinether und -ester, Lecithine, Ester und Ether von Zuckern oder Zuckeralkoholen mit Fettsäuren oder Fettalkoholen, Phospholipide und Sphingolipide, Sterole oder deren Ester und Ether, sowohl allein als auch in Form von deren Mischungen umfassen.

9. Arzneistoffträger nach Anspruch 7 oder 8, **dadurch gekennzeichnet, daß** die dispersionsstabilisierenden Substanzen Eilecithin, Soyalecithin oder hydrierte Lecithine, deren Mischungen oder der Mischung eines oder beider Lecithine mit einem oder mehreren Phospholipid-Komponenten, Cholesterol, Cholesterolpalmitat, Stigmasterin oder anderen Sterolen umfassen.

10. Arzneistoffträger nach einem der Ansprüche 1 bis 9, **dadurch gekennzeichnet, daß** er einen Zusatz von ionischen Emulgatoren zur Ladungsstabilisierung bezogen auf die ursprüngliche Zubereitung in einer Menge von 0,01% bis 10% (m/m) und insbesondere im Bereich von 0,05-2% (m/m) umfaßt.

11. Arzneistoffträger nach Anspruch 10, **dadurch gekennzeichnet, daß** er als geladene ionische Ladungsstabilisatoren Diacetylphosphat, Phosphatidylglycerol, gesättigte oder ungesättigte Fettsäuren, Natriumcholat, Natriumglycocholat, Natriumtaurocholat oder deren Mischungen und/oder Aminosäuren umfaßt.

12. Arzneistoffträger nach Anspruch 7, **dadurch gekennzeichnet, daß** er eine oder mehrere viskositätserhöhende Substanzen bezogen auf die ursprüngliche Zubereitung in einer Menge von 0,1% bis 10% (m/m) und insbesondere innerhalb eines Bereichs von 0,5% bis 5% (m/m) umfaßt.

13. Arzneistoffträger nach Anspruch 12, **dadurch gekennzeichnet, daß** er die viskositätserhöhenden Stoffe Cellulose, deren Ether und Ester, Polyvinylderivate, Alginate, Polyacrylate, Xanthane und/oder Pektine oder eine Mischungen derselben umfaßt.

14. Arzneistoffträger nach Anspruch 12 oder 13, **dadurch gekennzeichnet, daß** er außerdem einen oder mehrere Zucker und/oder einen oder mehrere Zuckeralkohole, insbesondere Glucose Mannose, Trehalose, Mannitol und/oder Sorbitol umfaßt.

15. Arzneistoffträger nach einem der Ansprüche 12 bis 14, **dadurch gekennzeichnet, daß** er (alleine oder in Kombination) Peptisatoren, insbesondere Natriumcitrat und/oder Natriumphosphate, umfaßt.

16. Arzneistoffträger nach einem der Ansprüche 1 bis 15, **dadurch gekennzeichnet, daß** die Partikel in destilliertem Wasser, in einer wäßrigen Lösung mit Zusätzen von Elektrolyten, Mono- und Disacchariden, Polyolen oder deren Mischungen oder in Flüssigkeiten, die mit Wasser mischbar sind, dispergiert sind, wobei als Additive Natriumchlorid, Mannose, Glucose, Fruktose, Xylose, Trehalose, Mannitol, Sorbitol, Xylitol und/oder Glycerol vorzugsweise in einer Menge von 0,1% bis 50% (m/m), und insbesondere im Bereich zwischen 1% und 30% (m/m) bezogen auf die ursprüngliche Zubereitung vorhanden sind.

17. Arzneistoffträger nach einem der Ansprüche 1 bis 16, **dadurch gekennzeichnet, daß** die Partikel lyophilisiert oder sprühgetrocknet sind oder durch andere Trocknungsprozesse in eine trockene Form überführt oder verarbeitet wurden.

18. Arzneistoffträger nach einem der Ansprüche 1 bis 17, **dadurch gekennzeichnet, daß** die Herstellung unter Ausschluß halogenierter organischer Lösungsmittel und bevorzugt unter Ausschluß von organischen Lösungsmitteln überhaupt erfolgt ist.

19. Arzneistoffträger nach einem der Ansprüche 1 bis 17, **dadurch gekennzeichnet, daß** Ciclosporin in natürlicher oder synthetischer Form, alleine als aktive Substanz (Wirkstoff) oder in Mischung mit anderen aktiven Substanzen (Wirkstoffen) vorhanden ist.

20. Arzneistoffträger nach Anspruch 19, **dadurch gekennzeichnet, daß** die aktive Substanz bzw. die aktiven Substanzen in den Partikeln fein dispergiert und/oder gelöst vorliegen und/oder auf der Oberfläche der Partikel adsorbiert vorliegen.

21. Arzneistoffträger nach einem der Ansprüche 1 bis 20, **dadurch gekennzeichnet, daß** 0,01% bis 70% Ciclosporin, vorzugsweise 1% bis 30% Ciclosporin, kalkuliert auf das Gewicht der Partikel (d.h. Lipid + Ciclosporin = 100%), vorhanden sind.

22. Arzneistoffträger nach einem der Ansprüche 1 bis 21, **dadurch gekennzeichnet, daß** er aus einer Mischung von Mono-, Di- und Triglyceriden der Palmitin- und Stearinsäure als Lipidmatrix, die mit 0,01% bis 70%, vorzugsweise 5% bis 30% Ciclosporin beladen ist und mit einer Mischung von Polyoxyethylenglycerolmonostearat und Natriumcholat stabilisiert, besteht.

23. Arzneistoffträger nach einem der Ansprüche 1 bis 21, **dadurch gekennzeichnet, daß** er aus einer Mischung von Mono-, Di- und Triglyceriden der Palmitin- und Stearinsäure als Lipidmatrix, die mit 5% bis 30% Ciclosporin beladen ist und mit Lecithinen, Poloxamer 188, einem Zuckeralkohol oder -ester oder -ether, Polysorbat 20, 40, 60 oder 80, Natriumcholat oder deren Mischungen stabilisiert ist, besteht.

24. Arzneistoffträger nach einem der Ansprüche 1 bis 23, **dadurch gekennzeichnet, daß** er in Form einer Endformulierung zur oralen und peroralen Anwendung als trockenes Pulver in Sachets zur Rekonstitution als Suspension vor dem Gebrauch, als Pellets, Granulat, Tablette, Brauseteblette, Kepsel und therapeutisches Arzneistofffreisetzungssystem, insbesondere als bioadhäsive Tablette vorliegt.

25. Arzneistoffträger nach einem der Ansprüche 1 bis 23, **dadurch gekennzeichnet daß** er in Form einer Endformulierung zur parenteralen Anwendung, insbesondere als Partikelsuspension oder zu größeren Freisetzungseinheiten verarbeitet, wie vorzugsweise Tabletten oder Zylindern zur Implantation vorliegt.

26. Arzneistoffträger nach einem der Ansprüche 1 bis 23, **dadurch gekennzeichnet, daß** er in Form einer Endformulierung zur dermalen Anwendung auf Haut und Schleimhäuten vorliegt, insbesondere in Form einer/eines Salbe, Creme, Paste, Stifts, Gels oder Lotion.

27. Arzneistoffträger nach einem der Ansprüche 1 bis 26 für die therapeutische Behandlung mit Ciclosporin-Formulierungen, die eine mittlere Blutspiegelkonzentration im Steady State Bereich von 300ng/ml bis über 1000ng/ml, vorzugsweise über 800ng/ml, insbesondere bis 900 ng/ml, bevorzugt von 400 ng/ml bis 800 ng/ml unter Abwesenheit hoher mittlerer initialer Blutspiegelkonzentrationen wesentlich über 1500 ng/ml, insbesondere über 1200 ng/ml, vorzugsweise über 1000 ng/ml und bevorzugter über 800 ng/ml erzeugt.

28. Arzneistoffträger nach Anspruch 27, der die mittlers Blutspiegelkonzentration im Steady State Bereich für einen verlängerten Zeitraum von mindestens 5 h, vorzugsweise mindestens 7 h, insbesondere 9 h erzeugt.

29. Ciclosporin Formulierung, die einen Arzneistoffträger nach Anspruch 1 umfaßt, wobei die Formulierung eine mittlere Blutspiegelkonzentration im Steady State Bereich von 300 ng/ml bis über 1000 ng/ml, vorzugsweise über 800 ng/ml, insbesondere bis 900 ng/ml, bevorzugt von 400 ng/ml bis 800 ng/ml unter Abwesenheit hoher mittlerer initialer Blutspiegelkonzentrationen wesentlich über 1500 ng/ml, insbesondere über 1200 ng/ml, vorzugsweise über 1000 ng/ml und bevorzugter über 800 ng/ml erzeugt.

30. Ciclosporin Formulierung nach Anspruch 29, wobei die Formulierung eine verlängerte mittlere Blutspiegelkonzentration im Steady State Bereich für einen verlängerten Zeitraum von mindestens 5 h, vorzugsweise mindestens 7 h, insbesondere 9 h erzeugt.

## Claims

1. Vehicle comprising solid lipid particles laden with ciclosporin or ciclosporin derivatives of natural and/or synthetic origin, wherein the particles have a particle diameter from 10 nm to 10 µm, were produced by high-pressure homogenization and are in the solid state at room temperature, and wherein the particles of the principal population have a mean PCS particle diameter (determined by photon correlation spectroscopy) in the range from 40 nm to 1000 nm

2. Vehicle according to Claim 1, comprising surfactant-containing or surfactant-free particles of a lipid or of a mixture of lipids which can be produced either by dispersing an internal phase (lipid phase) in a dispersion medium (water, aqueous solution or a water-miscible liquid) in molten or softened form, or by dispersing an internal phase (lipid phase) in a dispersion medium in a solid form, wherein the solid phase is finely comminuted prior to the dispersion process.

3. Vehicle according to Claim 2, **characterized in that** the particles of the principal population have a mean particle diameter in the range from 100 nm to 500 nm and **in that**, with suitably chosen process variables and additives, the PCS particle diameters are in the range from 40 nm to 100 nm.

4. Vehicle according to one of Claims 1 to 3, **characterized in that** the content of the internal phase (lipid phase) relative to the total formulation is in the range from 0.1% to 40% (m/m) and in particular in the range from 1% to 20% (m/m).

5. Vehicle according to one of Claims 1 to 4, **characterized in that** it has monoglycerides, diglycerides and/or triglycerides, fatty alcohols, their esters or ethers, waxes or lipid peptides or a mixture thereof, as the material of the particle matrix.

6. Vehicle according to one of Claims 1 to 5, **characterized in that** it contains glycerol trilaurate, glycerol myristate, glycerol palmitate, glycerol stearate and/or glycerol behenate, or a mixture thereof with mono-, di- and triglycerides, as glyceride mixture, in particular Imwitor 900, fatty alcohols, in particular cetyl alcohol and stearyl alcohol, and/or waxes, in particular cetyl palmitate and bleached beeswax.

7. Vehicle according to one of Claims 1 to 6, **characterized in that** it has an addition of one or more dispersion-stabilizing additives in an amount, relative to the total formulation, from 0.01% to 30% (m/m) and especially in the range from 0.5 to 10% (m/m).

8. Vehicle according to Claim 7, **characterized in that** the dispersion-stabilizing additives comprise substances from the series of the poloxamers, poloxamines, ethoxylated monoglycerides and diglycerides, ethoxylated lipids, ethoxylated fatty alcohols and alkyl phenols, ethoxylated fatty acid esters, polyglycerol ethers and esters, lecithins, esters and ethers of sugars or sugar alcohols with fatty acids or fatty alcohols, phospholipids and sphingolipids, sterols or their esters and ethers, both alone and as their mixtures.

9. Vehicle according to Claim 7 or 8, **characterized in that** the dispersion-stabilizing substances comprise egg lecithin, soya lecithin or hydrogenated lecithins, their mixtures or a mixture of one or both lecithins with one or more phospholipid components, cholesterol, cholesterol palmitate, stigmasterol or other sterols.

10. Vehicle according to one of Claims 1 to 9, **characterized in that** it comprises an addition of ionic emulsifiers for charge stabilization in an amount, relative to the original preparation, from 0.01% to 10% (m/m) and especially in the range 0.05-2% (m/m).

11. Vehicle according to Claim 10, **characterized in that** it comprises diacetyl phosphate, phosphatidyl glycerol, saturated or unsaturated fatty acids, sodium cholate, sodium glycocholate, sodium taurocholate or their mixtures and/or amino acids as charged ionic charge stabilizers.

12. Vehicle according to Claim 7, **characterized in that** it comprises one or more viscosity-increasing substances in an amount, relative to the original preparation, from 0.1% to 10% (m/m) and in particular within a range from 0.5% to 5% (m/m).

13. Vehicle according to Claim 12, **characterized in that** it comprises the viscosity-increasing substances cellulose, its ethers and esters, polyvinyl derivatives, alginates, polyacrylates, xanthans and/or pectins or mixtures thereof.

14. Vehicle according to Claim 12 or 13, **characterized in that** it additionally comprises one or more sugars and/or one or more sugar alcohols, especially glucose, mannose, trehalose, mannitol and/or sorbitol.

15. Vehicle according to one of Claims 12 to 14, **characterized in that** it comprises (alone or in combination) peptizing agents, in particular sodium citrate and/or sodium phosphates.

16. Vehicle according to one of Claims 1 to 15, **characterized in that** the particles are dispersed in distilled water, in an aqueous solution with additions of electrolytes, mono- and disaccharides, polyols or their mixtures or in liquids that are miscible with water, containing as additives sodium chloride, mannose, glucose, fructose, xylose, trehalose, mannitol, sorbitol, xylitol and/or glycerol preferably in an amount from 0.1% to 50% (m/m), and especially in the range from 1% to 30% (m/m) relative to the original preparation.

17. Vehicle according to one of Claims 1 to 16, **characterized in that** the particles are freeze-dried or spray-dried or have been converted or processed to a dry form by other drying processes.

18. Vehicle according to one of Claims 1 to 17, **characterized in that** production took place with the exclusion of halogenated organic solvents and preferably with the total exclusion of organic solvents.

19. Vehicle according to one of Claims 1 to 17, **characterized in that** ciclosporin in natural or synthetic form is present alone as active substance (active principle) or mixed with other active substances (active principles).

20. Vehicle according to Claim 19, **characterized in that** the active substance or the active substances is/are finely dispersed and/or dissolved in the particles and/or is/are adsorbed on the surface of the particles.

21. Vehicle according to one of Claims 1 to 20, **characterized in that** 0.01% to 70% ciclosporin, preferably 1% to 30% ciclosporin, calculated from the weight of the particles (i.e. lipid + ciclosporin = 100%), is present.

22. Vehicle according to one of Claims 1 to 21, **characterized in that** it consists of a mixture of mono-, di- and triglycerides of palmitic and stearic acid as lipid matrix, which is laden with 0.01% to 70%, preferably 5% to 30% ciclosporin and is stabilized with a mixture of polyoxyethyleneglycerol monostearate and sodium cholate.

23. Vehicle according to one of Claims 1 to 21, **characterized in that** it consists of a mixture of mono-, di- and triglycerides of palmitic and stearic acid as lipid matrix, which is laden with 5% to 30% ciclosporin and is stabilized with lecithins, Poloxamer 188, a sugar alcohol or sugar ester or sugar ether, Polysorbate 20, 40, 60 or 80, sodium cholate or their mixtures.

24. Vehicle according to one of Claims 1 to 23, **characterized in that** it is in the form of a final formulation for oral and peroral application as dry powder in sachets for reconstitution as a suspension before use, as pellets, granules, tablets, effervescent tablets, capsules and a therapeutic drug release system, in particular as bio-adhesive tablets.

25. Vehicle according to one of Claims 1 to 23, **characterized in that** it is in the form of a final formulation for parenteral application, in particular as a particle suspension or is processed into larger release units, preferably as tablets or cylinders for implantation.

26. Vehicle according to one of Claims 1 to 23, **characterized in that** it is in the form of a final formulation for dermal application on the skin and mucous membranes, in particular in the form of an ointment, cream, paste, stick, gel or lotion.

27. Vehicle according to one of Claims 1 to 26 for therapeutic treatment with ciclosporin formulations, which produces a mean blood level concentration in the steady state range from 300 ng/ml to over 1000 ng/ml, preferably over 800 ng/ml, in particular up to 900 ng/ml, preferably from 400 ng/ml to 800 ng/ml in the absence of high mean initial blood level concentrations substantially over 1500 ng/ml, especially over 1200 ng/ml, preferably over 1000 ng/ml and more preferably over 800 ng/ml.

28. Vehicle according to Claim 27, which produces the mean blood level concentration in the steady state range for an extended period of at least 5 h, preferably at least 7 h, in particular 9 h.

29. Ciclosporin formulation that comprises a vehicle according to Claim 1, wherein the formulation produces a mean blood level concentration in the steady state range from 300 ng/ml to over 1000 ng/ml, preferably over 800 ng/ml, in particular up to 900 ng/ml, preferably from 400 ng/ml to 800 ng/ml in the absence of high mean initial blood level concentrations substantially over 1500 ng/ml, especially over 1200 ng/ml, preferably over 1000 ng/ml and more preferably over 800 ng/ml.

30. Ciclosporin formulation according to Claim 29, wherein the formulation produces a prolonged mean blood level concentration in the steady state range for an extended period of at least 5 h, preferably at least 7 h, in particular 9 h.

## Revendications

1. Support pour médicament qui contient des particules lipidiques solides chargées de cyclosporine ou de dérivés de cyclosporine d'origine naturelle et/ou synthétique, où les particules présentent un diamètre de particule de 10 nm à 10 µm, ont été produites par homogénéisation à haute pression et se présentée un état solide à température ambiante, et les particules de la population principale possèdent un diamètre moyen de particule par PCS (déterminé au moyen d'une spectroscopie par corrélation des protons) dans la gamme de 40 nm à 1000 nm.

2. Support pour médicament selon la revendication 1, qui contient des particules, contenant un agent tensioactif ou sans agent tensioactif, d'un lipide ou d'un mélange de lipides que l'on peut produire par le fait que soit une phase interne (phase lipidique) est dispersée dans un milieu de dispersion (eau, solution aqueuse ou liquide miscible à l'eau) sous une forme fondue ou amollie ou bien une phase interne (phase lipidique) est dispersée dans un milieu de dispersion sous une forme solide et la phase solide est alors comminutée avant le processus de dispersion.

3. Support pour médicament selon la revendication 2, **caractérisé en ce que** les particules de la population principale possèdent un diamètre de particule dans la gamme entre 100 nm et 500 nm et **en ce que**, grâce à des paramètres de procédé et additifs choisis de façon appropriée, le diamètre PCS des particules se trouve entre 40 nm et 100 nm.

4. Support pour médicament selon l'une quelconque des revendications 1 à 3, **caractérisé en ce que** la teneur de la phase interne (phase lipidique) à rapportée à la formulation totale, se trouve dans la zone de 0,1% à 40% (m/m) et en particulier dans la zone comprise entre 1% et 20% (m/m).

5. Support pour médicament selon l'une quelconque des revendications 1 à 4, **caractérisé en ce qu'**il présente, en tant que matériau de la matrice de particules, des monoglycérides, diglycérides et/ou triglycérides, des alcools gras, leurs esters ou éthers, des cires ou des peptides lipidiques ou un mélange de ceux-ci.

6. Support pour médicament selon l'une quelconque des revendications 1 à 5, **caractérisé en ce qu'**il contient du trilaurate de glycérol, du miristate de glycérol, du palmitate de glycérol, du stéarate de glycérol et/ou du béhenate de glycérol ou un mélange de ceux-ci avec des mono-, di- et triglycérides en tant que mélange de glycérides, en particiulier Imwitor 900, des alcools gras, en particulier de l'alcool cétylique et de l'alcool stéarylique et/ou des cires, en particulier du palmitate de cétyle et de la cire d'abeilles blanchie.

7. Support pour médicament selon l'une quelconque des revendications 1 à 6, **caractérisé en ce qu'**il présente une addition d'un ou plusieurs additifs stabilisant la dispersion en une quantité rapportée à la formulation totale de 0,01% à 30% (m/m) et en particulier dans la gamme de 0,5 à 10% (m/m).

8. Support pour médicament selon la revendication 7, **caractérisé en ce que** les additifs stabilisant la dispersion contiennent des substances de la série des poloxamères, poloxamines, monoglycérides et diglycérides éthoxylés, lipides éthoxylés, alcools gras et alkyl phénols éthoxylés, esters d'acides gras éthoxylés, polyglycérine éthers et esters, lécithine, esters et éthers de sucres ou alcools de sucre avec des acides gras ou des alcools gras, phospholipides et sphingolipides, stérols ou leurs esters et éthers aussi bien seuls qu'également sous forme de leurs mélanges.

9. Support pour médicament selon l'une quelconque des revendications 7 ou 8, **caractérisé en ce que** les substances stabilisant la dispersion contiennent de la lécithine d'oeuf, de la lécithine de soja ou de la lécithine hydratée, leurs mélanges ou le mélange de l'une ou des deux lécithines avec un ou plusieurs composants de phospholipides, cholestérol, palmitate de cholestérol, stigmastérine ou autres stérols.

10. Support pour médicament selon l'une quelconque des revendications 1 à 9, **caractérisé en ce qu'**il contient une addition d'émulsionnants ioniques pour la stabilisation de la charge rapportée à la préparation d'origine en une quantité de 0,1% à 10% (m/m) et en particulier dans la gamme de 0,05-2% (m/m).

11. Support pour médicament selon la revendication 10, **caractérisé en ce qu'**il contient, en tant que stabilisants de la charge ionique, du diacétylphosphate, du phosphatidylglycérol, des acides gras saturés ou insaturés, du cholate de sodium, du glycolate de sodium, du taurocholate de sodium ou leurs mélanges et/ou acides aminés.

12. Support pour médicament selon la revendication 7, **caractérisé en ce qu'**il contient une ou plusieurs substances augmentant la viscosité, en se rapportant à la préparation d'origine, en une quantité de 0,1% à 10% (m/m) et en particulier dans une gamme de 0,5% à 5% (m/m).

13. Support pour médicament selon la revendication 12, **caractérisé en ce qu'**il contient les matières augmentant la viscosité cellulose, leurs éthers et esters, des dérivés de polyvinyle, alginates, polyacrylates, xanthane et/ou pectine ou un mélange de ceux-ci.

14. Support pour médicament selon la revendication 12 ou 13, **caractérisé en ce qu'**il contient, outre un ou plusieurs sucres et/ou un ou plusieurs alcools de sucre, en particulier glucose, mannose, tréhalose, mannitol et/ou sorbitol.

15. Support pour médicament selon l'une quelconque des revendications 12 à 14, **caractérisé en ce qu'**il contient (seuls ou en combinaison) des agents peptisants, en particulier du citrate de sodium et/ou du phosphate de sodium.

16. Support pour médicament selon l'une quelconque des revendications 1 à 15, **caractérisé en ce que** les particules sont dispersées dans de l'eau distillée, dans une solution aqueuse avec des additifs d'électrolytes, mono- et disaccharides, polyols ou leurs mélanges ou dans des liquides qui sont miscibles avec l'eau, et comme additifs, sont présents du chlorure de sodium, du mannose, du glucose, du fructose, du xylose, du tréhalose, du mannitol, du sorbitol, du xylitol et/ou du glycérol, avantageusement en une quantité de 0,1% à 50% (m/m) et en particulier dans une gamme comprise entre 1% et 30% (m/m) en se rapportant à la préparation d'origine.

17. Support pour médicament selon l'une quelconque des revendications 1 à 16, **caractérisé en ce que** les particules sont lyophilisées ou séchées par pulvérisation ou bien sont transformées ou traitées par un autre procédé de séchage en une forme sèche.

18. Support pour médicament selon l'une quelconque des revendications 1 à 17, **caractérisé en ce que** la production se produit à l'exclusion de solvants organiques halogénés et préférentiellement à l'exclusion de solvants organiques en général.

19. Support pour médicament selon l'une quelconque des revendications 1 à 17, **caractérisé en ce que** la cyclosporine est prescrite sous forme naturelle ou synthétique, seule en tant que substance active (agent actif) ou bien en mélange avec d'autres substances actives (agents actifs).

20. Support de médicament selon la revendication 19, **caractérisé en ce que** la substance active ou respectivement les substances actives dans les particules sont finement dispersées et/ou dissoutes et/ou se trouvent adsorbées à la surface des particules.

21. Support pour médicament selon l'une quelconque des revendications 1 à 20, **caractérisé en ce que** 0,01% à 70% de cyclosporine, avantageusement 1% à 30% de cyclosporine, en calculant sur le poids des particules (c'est-à-dire lipide + cyclosporine = 100%) sont présentes.

22. Support pour médicament selon l'une quelconque des revendications 1 à 21, **caractérisé en ce qu'**il se compose d'un mélange de mono-, di- et triglycérides des acides palmitique et stéarique, en tant que matrice lipidique, qui est chargée de 0,01% à 70%, avantageusement de 5% à 30% de cyclosporine et est stabilisée par un mélange de polyoxyéthylène glycérol monostéarate et de cholate de sodium.

23. Support pour médicament selon l'une quelconque des revendications 1 à 21, **caractérisé en ce qu'**il se compose d'un mélange de mono-, di- et triglycérides des acides palmitique et stéarique en tant matrice lipidique, qui est chargée de 50% à 30% de cyclosporine et stabilisée avec des lécithines du Poloxamer 188, un alcool de sucre ou ester ou éther, Polysorbat 20, 40, 60 ou 80 du cholate de sodium ou leurs mélanges.

24. Support pour médicament selon l'une quelconque des revendications 1 à 23, **caractérisé en ce qu'**il se présente sous la forme d'une formulation finale pour utilisation orale et perorale, en tant que poudre sèche en sachet pour une reconstitution en tant que suspension avant utilisation, en tant que boulettes, granulés, comprimés, comprimés effervescents, capsules et systèmes thérapeutiques de libération du médicament, en particulier en tant que comprimés bio-adhésifs.

25. Support pour médicament selon l'une quelconque des revendications 1 à 23, **caractérisé en ce qu'**il se présente sous la forme d'une formulation finale pour utilisation parentérale, en particulier en tant que suspension de particules ou traité en unités plus grandes de libération comme par exemple des comprimés ou cylindres pour une implantation.

26. Support pour médicament selon l'une quelconque des revendications 1 à 23, **caractérisé en ce qu'**il se présente sous la forme d'une formulation finale pour utilisation cutanée sur la peau et les muqueuses, en particulier sous la forme d'une pommade, une crème, une pâte, un bâtonnet, un gel ou une lotion.

27. Support pour médicament selon l'une quelconque des revendications 1 à 26, pour le traitement thérapeutique avec des formulations à base de cyclosporine, qui produit une concentration moyenne dans le sang, dans une zone à l'état stable, de 300ng/ml jusqu'à plus de 1000ng/ml, avantageusement plus de 800ng/ml, en particulier jusqu'à 900ng/ml, préférentiellement de 400ng/ml à 800ng/ml, en l'absence de concentrations moyennes initiales dans le sang plus élevées, essentiellement supérieures à 1500ng/ml, en particulier supérieures à 1200ng/ml, avantageusement supérieures à 1000ng/ml et préférentiellement supérieures à 800ng/ml.

28. Support pour médicament selon la revendication 27, qui produit la concentration moyenne dans le sang dans la zone à l'état stable pendant un espace prolongé de temps d'au moins 5 heures avantageusement au moins 7 heures, en particulier de 9 heures.

29. Formulation de cyclosporine, qui comporte un support pour médicament selon la revendication 1, où la formulation produit une concentration moyenne dans le sang dans une zone stable de 300ng/ml à plus de 1000ng/ml, avantageusement plus de 800ng/ml, en particulier jusqu'à 900ng/ml, préférentiellement de 400ng/ml à 800ng/ml en l'absence de concentrations initiales moyennes dans le sang supérieures, essentiellement au-delà de 1500ng/ml, en particulier au-delà de 1200ng/ml, avantageusement au-delà de 1000ng/ml et préférentiellement au-delà du 800ng/ml.

30. Formulation de ciclosporine selon la revendication 29, où la formulation produit une concentration moyenne prolongée dans le sang dans une zone stable pendant un intervalle de temps prolongé d'au moins 5 heures, avantageusement d'au moins 7 heures en particulier de 9 heures.
